# EUROPÄISCHE PATENTSCHRIFT

(11) **EP 2 068 788 B1**
(45) Veröffentlichungstag und Bekanntmachung des Hinweises auf die Patenterteilung: **08.08.2012**
(21) Anmeldenummer: 07818540.2
(22) Anmeldetag: 28.09.2007
(51) Int. Cl.: A61F 5/37

(54) **SICHERHEITSBANDAGE MIT EINER SICHERHEITSSCHLAUFE**
SAFETY BRACE WITH A SAFETY STRAP
BANDAGE DE SÉCURITÉ DOTÉ D'UNE BOUCLE DE SÉCURITÉ

(30) Priorität: 05.10.2006 DE 202006015369 U; 27.11.2006 DE 102006056169
(43) Veröffentlichungstag der Anmeldung: 17.06.2009
(73) Patentinhaber: Sànchez, Alexander, 21266 Jesteburg (DE)
(72) Erfinder: Sànchez, Alexander, 21266 Jesteburg (DE)
(74) Vertreter: Richter Werdermann Gerbaulet Hofmann
(86) Internationale Anmeldenummer: PCT/EP2007/008458
(87) Internationale Veröffentlichungsnummer: WO 2008/040496

(56) Entgegenhaltungen:
- DE-U1- 20 317 701
- DE-U1- 29 601 701
- DE-U1-202006 009 389
- DE-U1-202006 009 397
- GB-A- 1 132 546
- US-A- 3 817 245
- US-A- 4 132 229
- US-A- 5 558 102
- US-A- 5 660 445
- SEGUFIX BANDAGEN; DAS HUMANE SYSTEM GMBH & CO KG: "SEGUFIX-Akutfixierung"[Online] 8. September 2005 (2005-09-08), XP009092946 Gefunden im Internet: URL:http://web.archive.org/web/20050908111 650/www.segufix.de/Seiten/deutsch/akutfixi erung.html> [gefunden am 2007-12-05]

## Beschreibung

### Technisches Gebiet

Die Erfindung betrifft eine Sicherheitsbandage zur Begrenzung der Beweglichkeit eines auf einem Lager befindlichen Patienten bzw. zur Positionierung eines Patienten auf einem Lager mit einem Haltegurt, mit dem ein Leibgurt, der den Körper des Patienten im Bauchbereich umschließt, beweglich verbunden ist.

### Stand der Technik

Derartige Sicherheitsbandagen sind dem Fachmann in den unterschiedlichsten Ausführungsformen bekannt und geläufig und beispielsweise in der DE 296 01 701 und in der DE 20 2006 009 397 U1 beschrieben, letztere zeigt die Merkmale des Oberbegriffs des Anspruch 1.

Mit dem um den Bauchbereich des Patienten geschlungenen Leibgurt wird dessen Beweglichkeit soweit eingeschränkt, dass er das Lager, an dem der Leibgurt über einen Haltegurt befestigt ist, nicht entgegen dem Willen einer Aufsichtsperson verlassen kann. Um den Leibgurt, relativ zum Körper des Patienten an diesem zu fixieren, ohne den Leibgurt zu stramm anziehen zu müssen, was beispielsweise zu einer Beeinträchtigung der Bauchatmung des Patienten führen könnte, ist es aus der DE 203 17 701 U1 bekannt, an dem Leibgurt bzw. der Sicherhertsbandage über quer zu diesem ausgerichtete Beinhalterungsgurte zusätzliche Oberschenkelmanschetten anzuordnen. Dabei wird jeder der beiden Oberschenkel des Patienten von einer diesem zugeordneten Oberschenkelmanschette umgriffen, die mit geeigneten Verschlussmitteln zum Einstellen des Oberschenkelumfangs kelumfangs des Patienten versehen sind. Derartige Oberschenkelmanschetten gewährleisten, dass der Patient sich weder bewusst noch unbewusst aus der Sicherheitsbandage bzw. deren Leibgurt nach unten herauswinden und das Lager, beispielsweise ein Bett oder eine speziell vorgesehene Krankenliege, verlassen kann. Alternativ bzw. zusätzlich kann ein Gurt vorgesehen sein, der quer zum Leibgurt durch den Schritt des Patienten verläuft. Dieser kann jedoch bei einem Versuch des Herauswindens aus dem Gurt zu einer Genitalquetschung des Patienten führen. Ebenso sind Selbststrangulationen der Patienten mit diesem Gurt nicht ausgeschlossen.

Bei derartigen Sicherheitsbandagen mit Beinhalterungs- und Oberschenkelmanschetten ist es jedoch nicht ausgeschlossen, dass besonders ein gelenkiger Patient die Knie zum Oberkörper hin anzieht, so dass der relative Abstand zwischen dem Leibgurt und den Oberschenkelmanschetten verringert ist. Bei angezogenen Knien kann so der Patient die Oberschenkelmanschetten über seine Knie abstreifen, da die Beinhalterungsgurte lose sind.

### Darstellung der Erfindung, Aufgabe, Lösung, Vorteile

Es ist daher Aufgabe der Erfindung, eine gattungsgemäße Sicherheitbandage derart zu verbessern, dass ein Patient die Oberschenkelmanschetten nicht selbsttätig abstreifen kann, ohne ihn über Gebühr einzuengen, insbesondere soll ein seitliches Ziehen oder Drehen der Oberschenkelmanschetten, um diese abstreifen zu können, unterbunden werden, ohne dass die mit der Unterbringung und Betreuung der Patienten beauftragten Einrichtungen, komplette Fixierbandagen neu anschaffen müssen, sondern unter Nutzung bereits vorhandener SEGUFIX®-Leibgurte diese lediglich um den im folgenden beschriebene Nachrüstsatz ergänzen müssen.

Diese Aufgabe wird durch die Merkmale der Ansprüche 1 und 51 gelöst.

Der Kerngedanke der Erfindung ist, dass zur Anbringung weiterer Fixierelemente, bei denen zunächst an Schulter- und/oder Oberschenkelhalterungen in verschiedensten Ausführungsformen aber auch an Schlaufen zur lösbaren Anbringung anderer Fixierelemente gedacht ist, - deren feste Anbringung aus Sicherheitsgründen nur vom Fachmann, vorzugsweise vom Hersteller vorgenommen werden sollte -, hinreichend große und breite Flächen an einer Sicherheitsbandage fest oder lösbar angebracht werden. In einer hier verwendeten Terminologie werden solche Gegenstände als Fixiermittel bezeichnet, die der Patientenfixierung am Lager dienen.

Diese materiale Fläche kann an verschiedenen Stellen der Sicherheitsbandage angebracht sein. Auch ist es möglich, dass sich mehrere materiale Flächen an der Sicherheitsbandage befinden. Es ist daher von Vorteil, dass mindestens eine materiale Fläche über dem Leibgurt und/oder unter dem Haltegurt und/oder zwischen Leibgurt und Haltegurt fest oder lösbar mit diesem(n) verbunden ist.

Die Erfindung sieht vor, dass die Fläche Teil einer Sicherheitsschlaufe ist, die über den Leibgurt und den Haltegurt gesteckt ist, wobei die Sicherheitsschlaufe einseitig zum Kopf- oder Fußende oder beidseitig zum Kopf- und Fußende des Patienten mit der Fläche versehen ist, an denen Fixierelemente und/oder Schlaufen fest angebracht sind, wobei die Schlaufen der lösbaren Anbringung von Fixiermitteln dienen.

Nach Anbringung der Betthalterungen des Leibgurtes am Lager und Schließen des Leibgurtes ist die Sicherheitsschlaufe mit der Sicherheitsbandage derart verbunden, dass der Patient die Sicherheitsschlaufe und somit die daran befestigten Fixierelemente bzw. Befestigungsmittel zur lösbaren Anbringung weiterer Fixiermittel nicht entfernen kann. Ein solcher Verbund aus Sicherheitsschlaufe und daran angebrachten weiteren Fixierelementen, insbesondere Schulter- und/oder Oberschenkelhalterungen, ermöglicht den Pflegeeinrichtungen, vorhandene Sicherheitsbandagen, die den Patienten unter Umständen einer Gefahr durch Strangulation oder Genitalquetschungen aussetzen, wirtschaftlich zu ersetzen bzw. aufzurüsten, so dass der Patient gefährdungsfrei gelagert werden kann. Ein weiterer Vorteil dieser Erfindung liegt darin, die mittlerweile auf Grund der schlechter gewordenen Pflegesituation mit den Leib- und Haltegurt zusammenhaltenden gurtartigen Bändern zu groß gewordene Drehbeweglichkeit des Patienten, die mit obligaten Seitenbefestigungen eingeschränkt werden muss, von der erfindungsgemäßen Sicherheitsschlaufe allein eingeschränkt werden kann, insbesondere dann, wenn sie gegen Verrutschen gesichert wird oder mit Leib- und/oder Haltegurt fest verbunden wird.

Das Kernelement, die Sicherheitsschlaufe, besteht praktischer Weise aus ca. 36 cm breitem Gurtmaterial oder Material anderer Breite, das dergestalt zu einer Sicherheitsschlaufe miteinander verbunden, insbesondere zusammengenäht wird, dass eine Sicherheitsbandage umfasst werden kann und an deren einem Ende eine flächige Ausprägung entsteht, die hinreichend Platz bietet, um daran weitere Fixierelemente fest anzubringen und/oder um daran Schlaufen fest anzubringen, die der lösbaren Anbringung von Fixierelementen dienen. Dann könnte etwa wahlweise eine Schulter- oder eine Oberschenkelhalterung angebracht werden. Dabei kann die Fläche, wenn die Schlaufe über die Sicherheitsbandage gesteckt ist, zum Kopf- oder Fußende ausgerichtet werden, so dass die Anbringung insbesondere von Schulter- oder Oberschenkelhalterungen aber ggf. auch von anderen Fixiermitteln oder Schlaufen zu deren lösbaren Anbringung angebracht ist.

Eine solche Sicherheitsschlaufe kann dergestalt ringförmig aus einem Stück gestaltet sein, dass das eine Ende über das andere Ende hinausragt, wodurch eine aus einlagigem Material gebildete Fläche zur späteren Anbringung weiterer Fixierelemente und/oder Schlaufen für Fixierelemente entsteht.

Die Enden einer solchen aus einem Stück ringförmig gestalteten Sicherheitsschlaufe können aber auch bündig zusammengenäht werden, wodurch eine aus doppellagigem Material gebildete Fläche zur späteren Anbringung weiterer Fixierelemente und/oder Schlaufen für Fixierelemente entsteht.

Eine andere denkbare Variante wäre, dass sie außerhalb der Fläche zusammengenäht wird, also nicht am Rand, sondern mehr zur Mitte hin, etwa außerhalb der Flächen.

Die Sicherheitsschlaufe kann auch dergestalt gestaltet sein, dass sowohl an deren oberem als auch deren unterem Ende eine flächige Ausprägung entsteht, die beide hinreichend Platz bieten, um weitere Fixierelemente fest und/oder Schlaufen zur lösbaren Anbringung weiterer Fixierelemente anzubringen. Dabei wird die Sicherheitsschlaufe wie in den obigen Variationen geschlossen. Dann könnte sowohl eine Schulter- als auch eine Oberschenkelhalterung angebracht werden kann, was der Regelfall bei der Sicherheitsschlaufe sein wird.

Eine vorteilhafte Ausführungsform einer solchen Sicherheitsschlaufe wird dergestalt konstruiert, dass diese nicht aus einem Stück zu einem Ring sondern vielmehr aus zwei vorzugsweise rechteckigen Stücken zusammengenäht wird. Der Vorteil dieser vorteilhaften Ausführungsform besteht vor allem darin, dass das obere und untere Ende der Sicherheitsschlaufe zur Befestigung weiterer Fixierelemente nicht geknickt werden muss.

Außerdem wird die Produktion dahingehend vereinfacht, dass durchgängig von außen genäht werden kann. Für die Anwendung ist vorteilhaft, dass die Formgebung der Sicherheitsschlaufe mehr der einer flachen Sicherheitsbandage entspricht.

Dabei ist zunächst daran gedacht, dass diese beiden Stücke bündig miteinander verbunden, vorzugsweise zusammengenäht werden, sodass zwei doppellagige Flächen zur späteren Anbringung weiterer Fixierelemente und/oder Schlaufen für Fixierelemente entstehen.

Eine vorteilhafte Ausführungsform der Sicherheitsschlaufe besteht darin, dass nur eines der Stücke so lang ist und eine Fläche bildet, dass daran Fixiermittel angebracht werden. Das andere Stück kann so kurz gehalten werden, wie es zum Annähen am anderen Stück zur Gewährleistung der Schlaufenfunktion erforderlich ist, womit Material und Näharbeit gespart wird, da in den meisten Fällen kein doppellagiges Material erforderlich ist. Auf Grund ihrer Hauptanwendung, in Gebrauch befindliche Sicherheitsbandagen vom Typ SEGUFIX®-Standard aufzurüsten, an deren Leibgurt sich zwei kleine weiter zu verwendende Schlaufen zur lösbaren Anbringung einer Schulterhalterung befinden, wird eine vorteilhafte Ausführungsform der Sicherheitsschlaufe dergestalt gestaltet sein, dass diese nur über eine Fläche verfügt und auf deren gegenüber liegender Seite zwei Schlitze angebracht sind, durch die die Schlaufen der Sicherheitsbandage gesteckt werden, die für die lösbare Anbringung einer Schulterhalterung gedacht sind. An der Fläche wird vorzugsweise eine Oberschenkelhalterung fest oder über fest angebrachte Schlaufen lösbar angebracht.

Zum Schutz der Ränder, insbesondere der an den Schnittstellen, wird Einfassband aufgenäht, das die Ränder umschließt.

Die einstückig ausgebildete Sicherheitsschlaufe kann eine untere Hälfte aufweisen, die gegenüber einer oberen Hälfte der Sicherheitsschlaufe verbreitert ist.

Eine weitere vorteilhafte Ausgestaltung sieht vor, dass die Sicherheitsschlaufe mit Langlöchern zu versehen ist, durch die die Enden des Haltegurtes und/oder Leibgurtes gesteckt werden können, womit eine Sicherung gegen Verrutschen der Sicherheitsschlaufe entlang der Sicherheitsbandage gegeben ist.

Dabei genügt es, das untere der beiden die Sicherheitsschlaufe bildenden Stücke rechts und links mit einem Langloch zu versehen.

Alternativ kann das obere der beiden die Sicherheitsschlaufe bildenden Stücke rechts und links mit einem Langloch versehen sein.

Ebenso ist vorgesehen, sowohl das obere als auch das untere der beiden die Sicherheitsschlaufe bildenden Stücke jeweils rechts und links mit einem Langloch zu versehen.

Eine demgegenüber noch vorteilhaftere Ausgestaltung sieht vor, dass das untere Stück derart zum Lagerrand hin verbreitert wird, dass es dort gegen Verrutschen in Querrichtung zum Lager gesichert werden kann. Damit ist gleichzeitig sichergestellt, dass die Sicherheitsschlaufe die von den Leib- und Haltegurt zusammenhaltenden gurtartigen Bändern ermöglichte Drehbeweglichkeit auch ohne Seitenbefestigung weitestgehend unterbindet, also ohne dass noch Seitenbefestigungen notwendig wären, die anzubringen ohnehin vom Anwender vergessen werden könnten.

Dies kann dergestalt geschehen, dass an den äußersten Rändern des unteren Stückes Ösen und Langlöcher angebracht sind, durch die die Betthalterungen des Haltegurtes gesteckt werden, damit diese am Rand unterhalb des unteren Stückes der Sicherheitsschlaufe verlaufen, sodass deren Ösen auf die Schlösser der Betthalterung des Haltegurtes gesteckt werden können, was die Sicherheitsschlaufe gegen Verrutschen in Querrichtung zum Lager sichert.

Das untere Stück kann aber auch zu beiden Seiten hin mit solchen Langlöchern versehen sein, dass der Haltegurt durch das innere breite Langloch gesteckt wird, sodass er ab dort unterhalb des unteren Stücks der Sicherheitsschlaufe verläuft, damit anschließend die Betthalterung des Haltegurtes durch das schmalere äußere Langloch gesteckt wird, durch das nur die schmal ausgeführte Betthalterung passt, der breitere Haltegurt jedoch nicht, was die Sicherheitsschlaufe gegen Verrutschen in Querrichtung zum Lager sichert.

Hierzu ist es angebracht, den Haltegurt an seinen Enden mit nicht biegbaren Einlagen zu versteifen, sodass der Haltegurt nicht gewaltsam durch eines der äußeren Langlöcher gestoßen werden kann.

Dabei ist es von Vorteil, die Nähte an den flachen inneren Enden mit inneren Laschen dergestalt zu sichern, dass zwischen oberem und unterem Ende ein Stoffstück liegt, was dazu führt, dass im Belastungsfall nicht mehr nur vordringlich die innerste Fadenreihe einer Naht der Sicherheitsschlaufe vom Leibgurt belastet wird, wenn an der an der Sicherheitsschlaufe befestigten Schulter- oder Oberschenkelhalterung gezogen wird, sondern die Kraft vielmehr auf die Fadenreihen aller Nähte und den dazwischenliegenden Haftreibungsflächen der inneren Lasche verteilt wird.

Vorteilhafter ist es, die Nähte an den flachen inneren Enden mit Laschen zur Nahtsicherung dergestalt zu sichern, dass jeweils das obere und untere Ende mit einem Stoffstück verbunden ist, was dazu führt, dass im Belastungsfall, wenn an der an der Sicherheitsschlaufe befestigten Schulter- oder Oberschenkelhalterung gezogen wird, nicht mehr nur vordringlich die innerste Fadenreihe einer Naht der Schlaufe vom Leibgurt belastet wird, sondern die Kräfte vielmehr auf die Fadenreihen aller Nähte und den dazwischenliegenden Haftreibungsflächen der äußeren Lasche verteilt wird. Der größere Vorteil dieser Konstruktion liegt darin, dass die Kraftverteilung auf alle Nähte und von ihnen gebildeten dazwischenliegenden Haftreibungsflächen auch dann erfolgt, wenn versucht wird, die zusammengenähten Enden der Sicherheitsschlaufe auseinander zu reißen.

Dabei können die Endstücke des einen Stückes auch nach innen gestülpt werden, womit dieses Stück selbst die Laschenfunktion wahrnimmt.

Die Nähte dieser vorteilhaften Ausführungsform könnten wiederum mit Laschen zur Nahtsicherung gesichert werden, die dann außen liegen.

Weiterhin kann man eines oder beide der Stücke aus einem nicht bzw. nur wenig biegbaren Material herstellen, sodass dem Leibgurt eine stabilere Lagerung innerhalb der Sicherheitsschlaufe gegeben wird.

Dabei empfiehlt sich eine Verbindung der beiden Stücke mit Nieten.

Alternativ kann ein nicht bzw. nur wenig biegbares Material, dass naturgemäß weniger hautfreundlich ist, dergestalt in die Sicherheitsschlaufe eingenäht werden, dass dieses Material nicht mit dem Patienten in Berührung kommt.

Zur lösbaren Anbringung weiterer Fixierelemente können an den oberen und/oder unteren Flächen der Sicherheitsschlaufe weitere Schlaufen angebracht werden.

Oder es können die Fixierelemente auch direkt an den Flächen der Sicherheitsschlaufe oder aber auch direkt an der Sicherheitsschlaufe angebracht werden.

Um das Entweichen des Patienten nach oben zu unterbinden, ist am oberen Ende der Sicherheitsschlaufe eine Schulterhalterung lösbar anzubringen.

Im Rahmen der Erfindung ist es mit umfasst, dass die Schulterhalterung fest an der Sicherheitsschlaufe angebracht ist.

Dabei können an den rückwärtigen Schultergurten Schlaufenpaare zur Anbringung weiterer Bettverbindungsgurte, Taillengurte oder anderer Gurte angebracht sein.

Solche Gurte können auch fest mit den Schultergurten verbunden sein.

Die mit der Sicherheitsschlaufe fest oder lösbar verbundene Schulterhalterung kann dergestalt gestaltet sein, dass beide Enden des Taillengurtes jeweils mit beiden Schultergurten - diese wiederum miteinander verbindend - lösbar miteinander verbunden sind, womit erreicht ist, dass diese an beiden Schultergurten arretierten Taillengurtenden gegenüber Schulterhalterungen, deren eines Taillengurtende auf einer Seite lediglich durch eine Schlaufe eines Schultergurtes beweglich gesteckt wird, einen festeren Halt hat. Hierzu befinden sich an den Enden des Taillengurtes Ösen und an den Schultergurten Schlossaufnahmen.

Weiterhin werden aus demselben Grund am unteren Ende der Sicherheitsschlaufe Oberschenkelmanschetten über lösbar an der Sicherheitsschlaufe angebrachten Beinhalterungsgurten angebracht.

Im Rahmen der Erfindung ist es mit umfasst, dass die Beinhalterungsgurte fest an der Sicherheitsschlaufe angebracht sind.

Weiterhin ist es vorteilhaft, die Oberschenkelmanschetten über einen Quersteg miteinander zu verbinden, um auf diese Weise ein Entkommen des Patienten zusätzlich zu erschweren, indem ein seitliches voneinander ausrücken der Beine unterbunden wird.

Um das Abstreifen der Oberschenkelmanschetten wirkungsvoll zu unterbinden, bedarf es noch zweier die Oberschenkelmanschetten mit dem Leibgurt verbindender Längsgurte.

Diese sind an den Oberschenkelmanschetten fest vernäht oder mit Ösen versehen und somit lösbar anzubringen. Ebenfalls ist eine Schlaufe am Ende des Längsgurtes denkbar, die zur lösbaren Verbindung über die Oberschenkelmanschette gesteckt werden kann.

Das am Leibgurt lösbar anzubringende Ende kann mit Ösen, um es an am Leibgurt befindlichen Gurtschlössern der Firma SEGUFIX zu befestigen, oder mit Schlaufen, um diese über die Enden des Leibgurtes zu stecken, bevor der Leibgurt geschlossen wird, an den Schlaufen ggf. mit Ösen, um mit am Leibgurt befindlichen Gurtschlössern der Firma SEGUFIX gegen seitliches Ausrücken gesichert zu werden, versehen sein.

Die Längsgurte können auch an einer vom Fachmann zu bestimmenden Stelle mit den jeweiligen Enden des Leibgurtes und/oder mit den Oberschenkelmanschetten fest verbunden werden.

Eine zusätzliche Sicherheit gegen seitliches Ausrücken und somit gegen Abstreifen der Oberschenkelmanschetten gewährleistet der die Längsgurte mit dem jeweils darüberliegenden Beinhalterungsgurt und diese wiederum miteinander verbindende lange Quersteg.

Die letzte Sicherheit ist eine flächige oder anordnungsbedingte Flächenfunktion des Raumes zwischen erfindungsgemäßer Sicherheitsschlaufe, Beinhalterungsgurten und Querstegen, sodass der Patient nicht zwischen den Beinhalterungsgurten durchrutschen kann, in Verbindung mit einer Schulterhalterung.

So gewährleistet die Schulterhalterung, dass der Patient sich nicht nach oben aus der Sicherheitsbandage herauswinden kann, die Oberschenkelmanschetten wiederum, dass sich der Patient nicht nach unten herauswinden kann, wobei der Vorteil dieser Erfindung darin liegt, dass die Genitalien des Patienten nicht gequetscht werden können, da der sonst bei auf dem Stand der Technik befindlichen Sicherheitsbandagen benötigte Schrittgurt zur Verhinderung des Rutschens nach unten hier nicht benötigt wird. Auch gewährleisten die eng aber nicht fest anzulegenden Oberschenkelmanschetten einen sicheren Halt des Patienten im Sicherheitsgurt, da diese den Patienten festhalten und doch mit dessen Bewegungen mitgehen.

Damit der Patient die Oberschenkelhalterungen nicht abstreifen kann, ist eine materiale Fläche zwischen Sicherheitsschlaufe, Beinhalterungsgurten und Querstegen erforderlich, sodass der Patient nicht zwischen den Beinhalterungsgurten hindurchrutschen kann.

Zieht der Patient beispielsweise ein Bein an, um dessen Oberschenkelmanschette abstreifen zu können, so bewirkt die materiale Fläche, dass dieses Anziehen bzw. Anwinkeln nur bis zu einem gewissen Grad möglich ist, da über die materiale Fläche bzw. den weiteren Quersteg auch das andere Bein über die materiale Fläche bzw. den Quersteg, an der bzw. dem beide Oberschenkelmanschetten angebracht sind, in Richtung zum Körper hin mitgezogen wird. Werden aber beide Beine gleichzeitig angezogen, so ist das Abstreifen der Oberschenkelmanschetten nicht möglich, da bei angezogenen Beinen die Schulterhalterung über die materiale Fläche einen sanften Druck auf die Oberschenkel und das Gesäß des Patienten ausüben und dabei den Abstand der Oberschenkelmanschetten zu den Knien quasi selbstregulierend vergrößern, was einer selbstregulierenden Abstandsfunktion gleichkommt. Die materiale Fläche muss dabei hinreichend breit gewählt sein, dass der Patient mit seinem Gesäß nicht an ihr vorbeirutschen kann, um den selbstregulierenden Mechanismus zu umgehen.

Eine weitere vorteilhafte Ausgestaltung der Erfindung besteht darin, dass die Oberschenkelhalterung mit einem eigenen Bettverbindungsgurt versehen ist, sodass diese zusätzlich am Lager befestigt werden kann, was ein Drehen des Patienten in der Vertikalachse verhindert.

Der Verbindungsgurt an der Oberschenkelhalterung kann auch dergestalt nur mit Ösen versehen und bemessen sein, dass dessen freie Enden an den Gurtschlössern des Leibgurtes befestigt werden können. Damit ist der Patient am Drehen um die Vertikalachse gehindert, hat jedoch mit den Beinen bzw. Oberschenkeln mehr Bewegungsfreiheit.

Statt mit Ösen können die Betthalterungen der Oberschenkelhalterung auch dergestalt mit den Betthalterungen des Haltegurtes verbunden sein, dass an den Enden der Betthalterungen der Oberschenkelmanschetten jeweils eine Schlaufe angebracht ist, durch die die Betthalterung des Haltegurtes durchgesteckt wird, bevor sie am Bettrahmen befestigt wird.

Ebenso ist es vorgesehen, die Betthalterungen der Oberschenkelhalterung ohne weitere Verschlussmittel direkt mit den Betthalterungen des Haltegurtes oder mit dem Haltegurt selbst fest zu verbinden, etwa zu vernähen oder zu vernieten.

Es ist im Rahmen der Erfindung mit umfasst, dass die Schulter- und Oberschenkelhalterung in beliebigen Kombinationen gemeinsam oder allein an der Sicherheitsschlaufe lösbar, fest oder lösbar und fest angebracht sind.

Aus dem ursprünglichen Verwendungszweck der Sicherheitsschlaufe heraus, SEGUFIX®-Leibbandagen zu einer auch unter den schlechter gewordenen Pflegebedingungen gefährdungsfreien Sicherheitsbandage aufzurüsten, wird an einem Ende eine Schulterhalterung fest oder lösbar angebracht und am anderen Ende eine Oberschenkelhalterung fest oder lösbar angebracht.

Es ist im Rahmen der Erfindung mit umfasst, dass die Sicherheitsschlaufe mit der Sicherheitsbandage fest verbunden werden kann, etwa vernäht oder mit Nieten, was aus Sicherheitsgründen nur vom Hersteller vorgenommen werden sollte.

Dabei können auch die dann unnötig gewordenen Haltegurt und Leibgurt verbindenden gurtartigen Bänder entfernt werden, um dann Leibgurt, Haltegurt und Sicherheitsschlaufe miteinander fest zu verbinden. Damit wäre bei hinreichend breiter Wahl der Verbindungsstelle zwischen Leibgurt und Haltegurt eine starre Fixierbandage erreicht, die gleichzeitig sicherstellt, dass die von den Leib- und Haltegurt zusammenhaltenden gurtartigen Bändern ermöglichte Drehbeweglichkeit auch ohne Seitenbefestigungen weitestgehend unterbunden wird, also ohne dass noch Seitenbefestigungen notwendig wären, die anzubringen ohnehin vergessen werden könnte.

Um die Funktion der Bänder beizubehalten ist es denkbar, nur das obere Stück der Sicherheitsschlaufe mit dem Leibgurt fest zu verbinden.

Ebenso kann das untere Stück der Sicherheitsschlaufe nur mit dem Haltegurt fest verbunden werden.

Im Rahmen der Erfindung ist mit umfasst, dass nicht die materiale Fläche und/oder die Sicherheitsschlaufe als ganzes sondern nur eines der Stücke unter Fortlassung des anderen Stückes auf dem Leibgurt, zwischen Leib- und Haltegurt oder unter den Haltegurt unter Fortlassung oder Beibehaltung der Leibgurt und Haltegurt zusammenhaltenden gurtartigen Bänder angenäht wird. Das eine Stück ist dabei hinreichend lang, so dass an dessen einem unteren oder einem oberen oder beiden Enden Fixiermittel und/oder Schlaufen zur lösbaren Anbringung von Fixiermitteln angebracht werden können.

Die oben beschriebenen Nachrüstmöglichkeiten können auch direkt vom Hersteller im Zuge der Neuproduktion vorgenommen werden. Dann ist es nicht mehr erforderlich, dass der Anwender in seinem Hause vorhandene Sicherheitsbandagen zur Nachrüstung an den Hersteller oder einen akkreditierten Fachbetrieb sendet, sondern er bezieht direkt von Hersteller ein den aktuellen Sicherheitsanforderungen genügendes Produkt.

Für den Verschluss der an der Sicherheitsschlaufe anzubringenden Fixiermittel mit Ösen und zugehörigen Schlossaufnahmen sind insbesondere Gurtschlösser der Firma SEGUFIX geeignet und aus Kompatibilitätsgründen und daraus möglicherweise nach dem Medizinproduktegesetz resultierenden haftungsrechtlichen Folgen auf der Betreiberseite ausschließlich zu empfehlen.

Für den Verschluss der an der Sicherheitsschlaufe anzubringenden Fixiermittel sind je nach Patientenbild neben Gurtschlössern der Firma SEGUFIX ebenfalls Klettverschlüsse, Gürtelschnallen und dergleichen geeignet.

Zudem sieht die Erfindung einen Nachrüstsatz für die Sicherheitsbandage gemäß anspruch 51 vor. Die Sicherheitsschlaufe des Nachrüstsatzes ist dabei gemäß einem der Ansprüche 2 bis 49 ausgebildet.

### Kurze Beschreibung der Zeichnungen

Nachstehend wird die Erfindung anhand von Zeichnungen näher erläutert. Es zeigen in schematischen Darstellungen:
- Fig. 1: einen Schnitt einer ringförmig gestalteten Sicherheitsschlaufe mit Flächen zur festen Anbringung von Fixiermitteln gemäß der Erfindung;
- Fig. 2: einen Schnitt einer aus zwei gleichlangen rechteckigen Stücken zusammengenähten Sicherheitsschlaufe mit Flächen und daran befestigten Schlaufen zur lösbaren Anbringung von Fixiermitteln;
- Fig. 3: eine perspektivische Sicherheitsschlaufe mit fest angebrachten Schlaufen zur lösbaren Anbringung von Fixiermitteln, beispielsweise einer Schulterhalterung und einer Fläche mit fest angebrachter Oberschenkelhalterung;
- Fig. 4: einen Schnitt einer ringförmig gestalteten Sicherheitsschlaufe, deren Enden nicht bündig aufeinander genäht sind, sodass auf einer Seite die zusätzlichen Fixiermittel auf einlagigem Material angebracht werden;
- Fig. 5: einen Schnitt einer aus zwei unterschiedlich langen rechteckigen Stücken zusammengenähten Sicherheitsschlaufe mit Flächen aus einlagigem Material;

- Fig. 6: einen Schnitt einer aus zwei unterschiedlich langen rechteckigen Stücken zusammengenähten Sicherheitsschlaufe mit Flächen aus einlagigem Material,
- Fig. 7: einen Schnitt einer ringförmig gestalteten Sicherheitsschlaufe mit nur einer einlagigen Fläche;
- Fig. 8a-b: einen Schnitt sowie eine Vorderansicht einer ringförmig gestalteten Sicherheitsschlaufe mit nur einer doppellagigen Fläche;
- Fig. 9a-b: eine Aufsicht sowie einen Schnitt entlang der Schnittlinie II-II aus Fig. 9b einer aus zwei gleichlangen Stücken gestalteten Sicherheitsschlaufe mit zwei doppellagigen Flächen zur festen Anbringung von Fixiermitteln, wobei das untere und obere Teil der Sicherheitsschlaufe mit Langlöchern und versehen ist, durch die die Enden des Haltegurtes und des Leibgurtes zur Sicherung gegen Verrutschen gesteckt werden;
- Fig. 10a-b: eine Aufsicht sowie einen Schnitt entlang der Schnittlinie II-II aus Fig. 10b einer aus zwei gleichlangen Stücken gestalteten Sicherheitsschlaufe mit zwei doppellagigen Flächen zur festen Anbringung von Fixiermitteln, wobei das untere Teil der Sicherheitsschlaufe so weit verbreitert ist, dass durch die Langlöcher an deren Enden die Betthalterung des Haltegurtes zur Sicherung gegen Verrutschen gesteckt werden;
- Fig. 11: eine Schulterhaltung mit einem offenen und einem geschlossenen Ende zur festen Anbringung an der Sicherheitsschlaufe.

### Bester Weg zur Ausführung der Erfindung

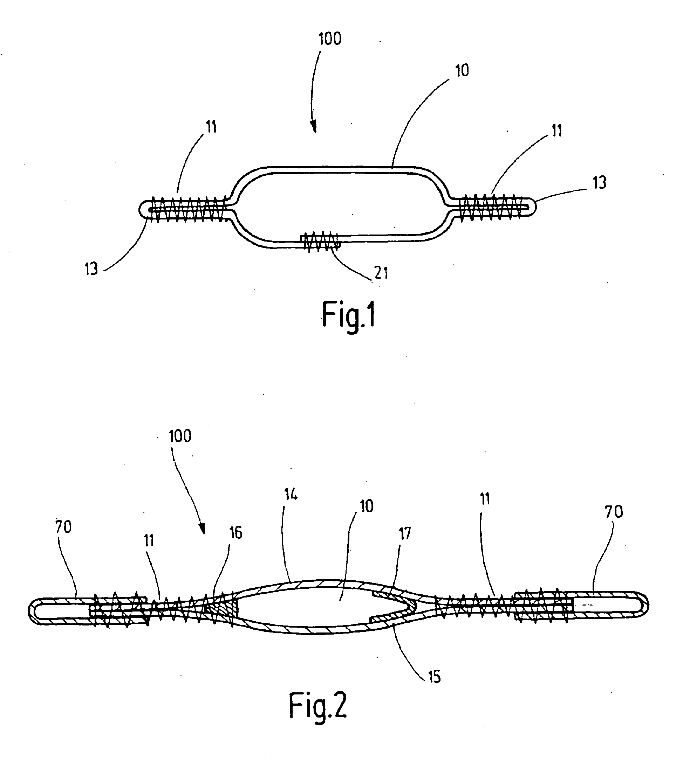

Fig. 1 zeigt eine erfindungsgemäße Sicherheitsschlaufe, die mit dem Bezugszeichen 10 versehen ist.

Die ringförmig gestaltete Sicherheitsschlaufe 10 weist Flächen 11 zur festen Anbringung von Schlaufen und/oder Fixiermitteln auf. Im Schnitt ist zu sehen, dass der die Sicherheitsschlaufe 10 bildende Gurt an einer die beiden Enden des Gurtes überlappenden Stelle 21 festgenäht ist. Beiderseitig der Schlaufe 10 verläuft ein Knick 13, der den Abschluss der Fläche 11 darstellt, die durch Zusammennähen der Sicherheitsschlaufe 10 an ihren Enden entsteht.

Aus Fig. 2 geht eine aus zwei vorzugsweise rechteckig geformten Stücken 14 und 15 zusammengenähte Sicherheitsschlaufe 10 mit Flächen 11 zur festen Anbringung von Schlaufen und/oder Fixiermitteln hervor. Im Schnitt ist zu sehen, dass die die Sicherheitsschlaufe 10 bildende Stücke 14, 15 an deren beiden Enden jeweils die Fläche 11 bildend zusammengenäht sind. Die inneren Nahtstellen der Sicherheitsschlaufe 10 sind jeweils mit einer klotzartigen und einer laschenartigen inneren Lasche 16, 17 zur Nahtsicherung gesichert. Weiterhin sind an den Flächen 11 Schlaufen 70 und im Schnitt nicht sichtbare Schlaufen 71 zur lösbaren Anbringung weiterer Fixiermittel bzw. Gürtel angebracht.

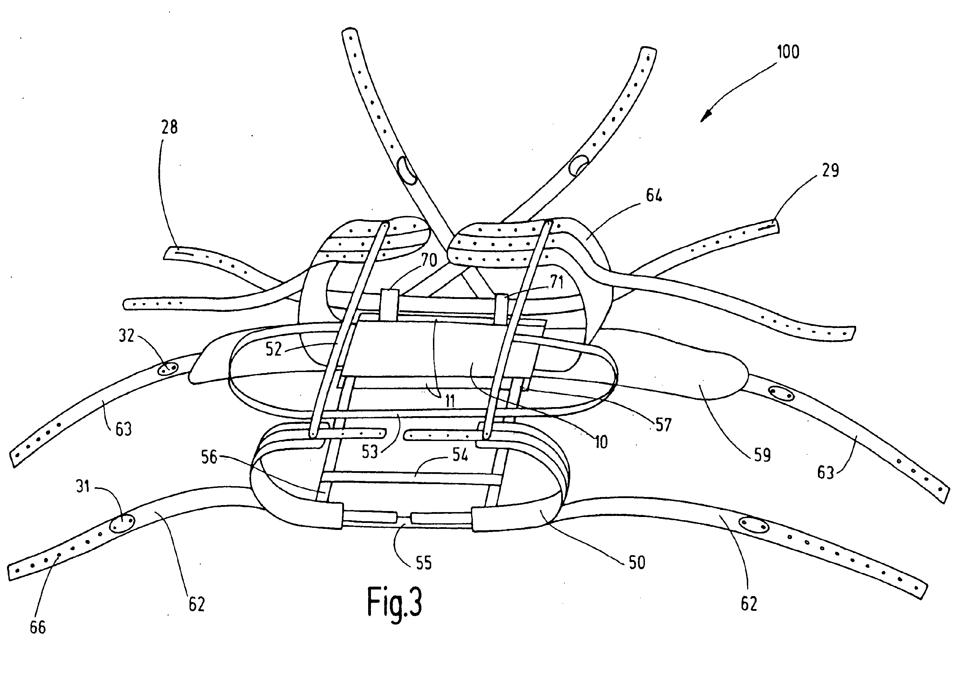

Fig. 3 zeigt die Sicherheitsschlaufe 10 der Sicherheitsbandage 100 im Verbund mit einer Schulterhalterung, deren Taillengurtenden 28 und 29 durch die an der oberen Fläche 11 fest angebrachten Schlaufen 70 und 71 der Sicherheitsschlaufe 10 gesteckt sind, und die somit lösbar angebracht sind, sowie zwei fest an der unteren Fläche 11 über die Beinhalterungsgurte 56 und 57 angebrachten Oberschenkelmanschetten 50, komplett mit fest am Leibgurt 64 angebrachten Längsgurten 52, und Querstegen 53, 54 und 55. Die unterhalb von Leibgurt 64 und Sicherheitsschlaufe 10 einen "Unterbau" bildenden Gurte sind dergestalt angeordnet, dass der Patient weder zwischen den Beinhalterungsgurten 56 und 57 hindurchrutschen noch die Oberschenkelmanschetten 50 in einem hinreichenden Maße seitlich ausrücken kann, um die Oberschenkelmanschetten 50 abzustreifen, sodass der Patient vor dem strangulationsgefährlichen Hinunterrutschen bewahrt wird, ohne dass ein genitalquetschender Schrittgurt erforderlich wäre. Weiterhin sind an den Oberschenkelmanschetten Betthalterungen 62 mit hinreichend vielen Ösen 66 angebracht, dass die Betthalterungen 62 wahlweise mit einem in der eigenen Tasche 31 befindlichen Gurtschloss 25 am Lager oder mit einer der Ösen 66 am in der Tasche 32 der Betthalterung 63 des Haltegurtes 59 befindlichen Gurtschloss 25 befestigt werden kann. Die hier nicht dargestellte lösbare Variante des "Unterbaus" mit Oberschenkelmanschetten 50 wäre dergestalt, das am unteren Ende der Sicherheitsschlaufe 10 zwei weitere Schlaufen 70, 71 angebracht sind, durch die ein mit dem Lager zu verbindender Bettverbindungsgurt gesteckt wird, an dem die Beinhalterungsgurte 56 und 57 fest angebracht sind. Die Längsgurte 52 wären wiederum lösbar am Leibgurt 64 anzubringen.

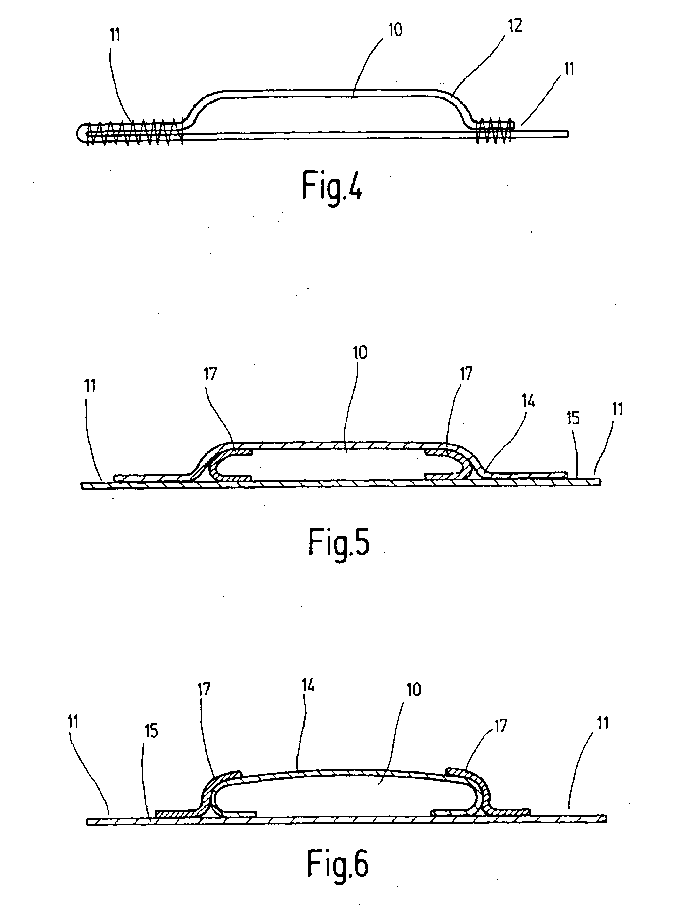

Aus Fig. 4 geht eine ringförmig gestaltete Sicherheitsschlaufe 10 mit Flächen 11 zur festen Anbringung von Schlaufen und/oder Fixiermitteln hervor. Im Schnitt ist zu sehen, dass die Sicherheitsschlaufe 10 an einer beide Enden überlappenden Stelle festgenäht ist. Dabei ist nur das linke, doppellagige Ende der Sicherheitsschlaufe 10 geknickt, wo es eine Fläche 11 bildend zusammengenäht ist. Rechts hingegen geht das untere Ende über das obere hinaus, sodass rechts von der Nahtstelle eine Fläche 11 aus einlagigem Material zur Anbringung weiterer Fixierelemente verbleibt.

Fig. 5 zeigt eine aus zwei unterschiedlichen langen rechteckigen Stücken 14, 15 zusammengenähte Sicherheitsschlaufe 10 mit Flächen 11 zur festen Anbringung von Schlaufen und/oder Fixiermitteln, wobei das obere Stück 14 verkürzt ausgeführt ist, sodass die Flächen 11 beiderseits aus einlagigem Material gebildet sind. Die inneren Nahtstellen der Sicherheitsschlaufe 10 sind beiderseits jeweils mit laschenartigen inneren Laschen 17 zur Nahtsicherung gesichert.

Aus Fig. 6 geht eine aus zwei unterschiedlichen langen rechteckigen Stücken 14, 15 zusammengenähte Sicherheitsschlaufe 10 mit den Flächen 11 zur festen Anbringung von Schlaufen und/oder Fixiermitteln hervor, wobei das obere Stück 14 verkürzt ausgeführt ist, sodass die Flächen 11 beiderseits aus einlagigem Material gebildet sind. Im Gegensatz zu Fig. 5 sind die äußeren Stellen des kürzeren Stückes 14 nach innen gebogen. Die äußeren Nahtstellen der Sicherheitsschlaufe 10 sind beiderseits jeweils mit laschenartigen äußeren Laschen 17 zur Nahtsicherung gesichert.

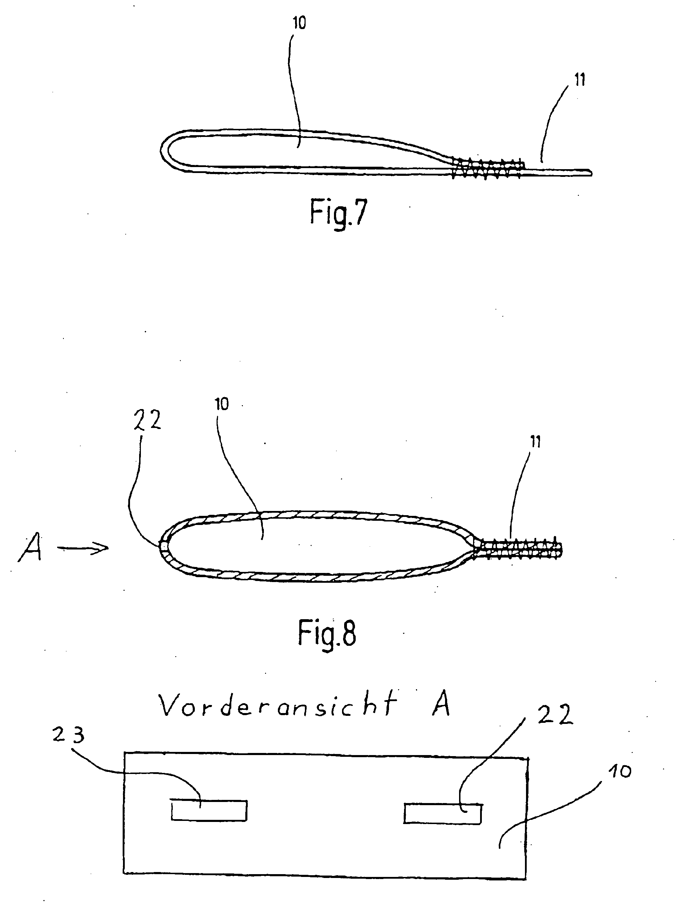

Fig. 7 zeigt eine ringförmig gestaltete Sicherheitsschlaufe 10 mit nur einer Fläche 11 zur festen Anbringung von Schlaufen und/oder Fixiermitteln. Im Schnitt ist zu sehen, dass der die Sicherheitsschlaufe 10 bildende Gurt an einer beide Enden überlappenden Stelle festgenäht ist. Links ist die Sicherheitsschlaufe 10 einfach nur geschlossen, ohne eine Fläche 11 zu bilden. Auf dieser Seite könnten ebenso wie in Fig. 8 Schlitze zum Durchlass der zwei am Leibgurt (64) der Sicherheitsbandage (100) vom Typ SEGUFIX®-Standard zur lösbaren Anbringung einer Schulterhalterung gedachten angebrachten Schlaufen sein, so dass die lösbare Schulterhalterung trotz übergesteckter Sicherheitsschlaufe (10) an den dafür vorgesehenen Schlaufen der Sicherheitsbandage (100) angebracht werden kann. Rechts hingegen geht das untere Ende über das obere hinaus, sodass rechts von der Nahtstelle eine Fläche 11 aus einlagigem Material zur Anbringung weiterer Fixierelemente verbleibt. Ebenso ist eine aus doppellagigem Material gebildete Fläche 11 denkbar, wenn beide Enden der Sicherheitsschlaufe 10 bündig zusammengenäht werden.

In Fig. 8 ist eine ringförmig gestaltete Sicherheitsschlaufe 10 mit nur einer Fläche 11 zur festen Anbringung von Schlaufen und/oder Fixiermitteln gezeigt. Im Schnitt ist zu sehen, dass der die Sicherheitsschlaufe 10 bildende Gurt an dessen Enden bündig festgenäht ist. Im Gegensatz zu Fig. 7 ist die Sicherheitsschlaufe 10 links nicht einfach nur geschlossen, ohne eine Fläche 11 zu bilden. Vielmehr befinden sich dort zwei Schlitze (22, 23), die dem Durchlass der zwei am Leibgurt (64) der Sicherheitsbandage (100) vom Typ SEGUFIX®-Standard zur lösbaren Anbringung einer Schulterhalterung gedachten angebrachten Schlaufen dienen, so dass die lösbare Schulterhalterung trotz übergesteckter Sicherheitsschlaufe (10) an den dafür vorgesehenen Schlaufen der Sicherheitsbandage (100) angebracht werden kann. Rechts hingegen bilden eine aus doppellagigem Material gebildete Fläche 11 die zusammengenähten Enden zur Anbringung weiterer Fixierelemente, insbesondere einer Oberschenkelhalterung. Ebenso ist eine aus einlagigem Material gebildete Fläche 11 denkbar, wenn eines der Enden der Schlaufe 10, wie in Fig. 7 gezeigt, über das andere Ende hinausragt.

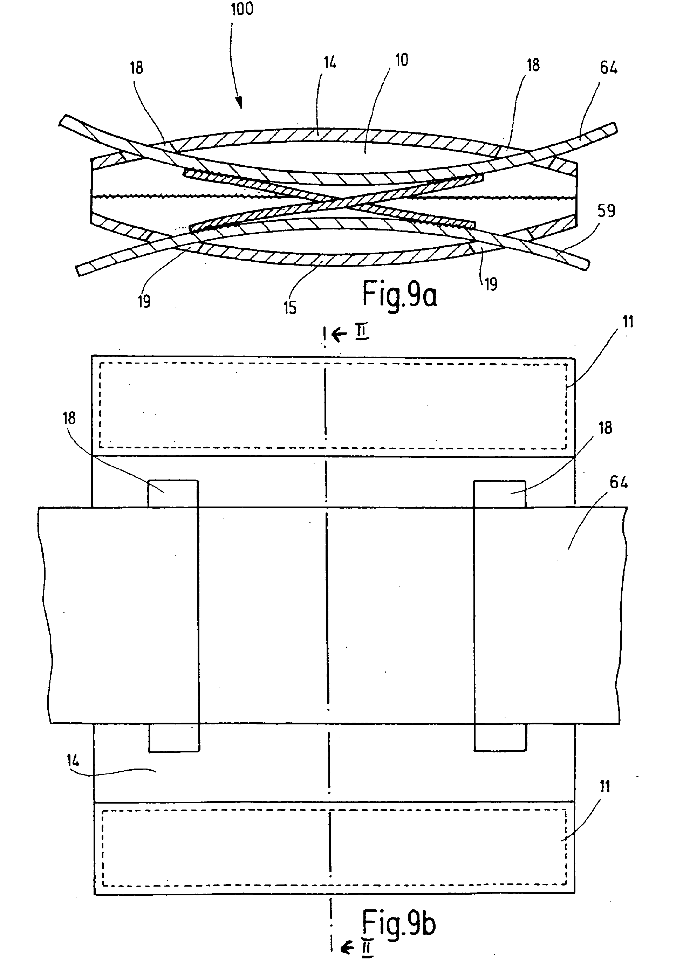

Fig. 9a und Fig. 9b in Draufsicht zeigen eine aus zwei gleichlangen Stücken 14, 15 gestaltete Schlaufe 10 mit zwei doppellagigen Flächen 11 zur festen Anbringung von Schlaufen und/oder Fixiermitteln. Der Haltegurt 59 bzw. Leibgurt 64 der Sicherheitsbandage 100 werden dabei nicht einfach durch die Sicherheitsschlaufe 10 gesteckt. Stattdessen werden die Enden des Leibgurtes 64 durch die oberen Langlöcher 18 des oberen Stückes 14 und die Enden des Haltegurtes 59 durch die unteren Langlöcher 19 gesteckt. Damit ist einem Verrutschen der Sicherheitsschlaufe 10 vorgebeugt.

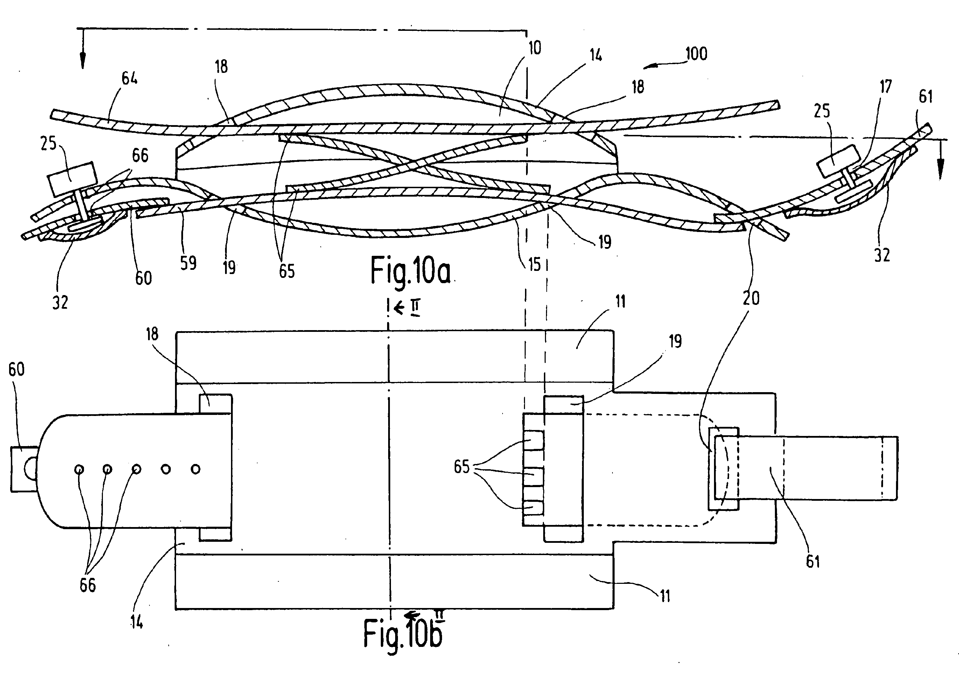

In Fig. 10a und in Fig. 10b in Draufsicht ist ein aus zwei gleichlangen Stücken gestaltete Sicherheitsschlaufe 10 mit zwei doppellagigen Flächen 11 zur festen Anbringung von Schlaufen und/oder Fixiermitteln dargestellt, wobei das untere Teil 15 der Sicherheitsschlaufe 10 so weit verbreitert ist, dass durch die Langlöcher 20 an deren Enden die Betthalterung des Haltegurtes 59 zur Sicherung gegen Verrutschen gesteckt werden. Dabei ist das untere Stück 15 der Sicherungsschlaufe 10 so weit verbreitert, dass es quasi wie ein zweiter "Haltegurt unter dem Haltegurt 59" liegt. Dabei kann das untere Stück 15, wie links dargestellt, von einer Öse 66 oder, wie rechts dargestellt, von der Rundung des Haltegurtes 59 gehalten werden, wobei rechts das Endstück des verbreiterten unteren Stücks 15 sowohl über als auch unter dem Haltegurt 59 enden kann. Um die Position zu stabilisieren, können am unteren Stück 15 zusätzlich zu den hier dargestellten weitere Langlöcher 19 paarweise am unteren Stück 15 angebracht werden, durch die der Haltegurt 59 durchgesteckt wird, sodass der Haltegurt 59 und das verbreiterte untere Stück 15 auch in Querrichtung nicht gegeneinander verrutschen.

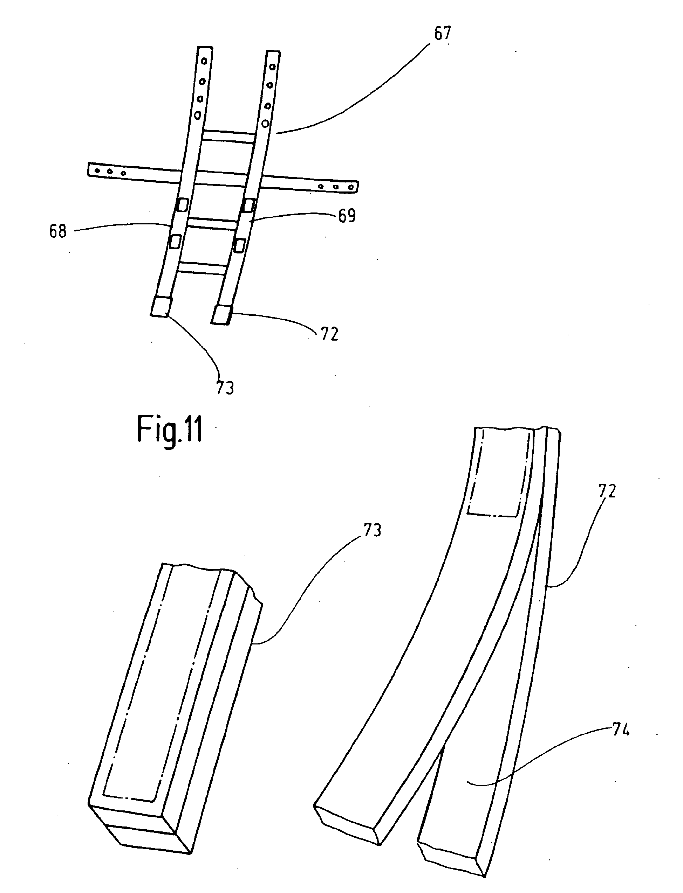

Aus Figur 11 geht eine Schulterhalterung 67 mit Schultergurten 68, 69 hervor, die über ein offenes Ende 72 und ein geschlossenes Ende 73 zur festen Abringung der Sicherheitsschlaufe 10 verfügt. Für die Anbringung an der Sicherheitsschlaufe 10 kann das geschlossene Ende 73 an einer Seite direkt mit der Sicherheitsschlaufe 10 vernäht werden. Denkbar ist auch, dass die Sicherheitsschlaufe 10 bei einem offenen Ende 72 Aufnahme in der Öffnung 74 des offenen Endes 72 findet, um sie dann beidseitig mit dem offenen Ende 72 zu vernähen.

### Bezugszeichenliste:

- 100: Sicherheitsbandage
- 10: Sicherheitsschlaufe
- 11: Fläche
- 12: Schulterhalterung
- 13: Knick
- 14: oberes Stück
- 15: unteres Stück
- 16: Lasche als Klotz
- 17: Lasche
- 18: obere Langlöcher
- 19: untere Langlöcher
- 20: Langloch
- 21: Stelle
- 22: Schlitz für Schlaufe für Schulterhalterung
- 23: Schlitz für Schlaufe für Schulterhalterung
- 25: Schloss
- 28: Taillengurtende
- 29: Taillengurtende
- 31: Tasche der Betthalterung der Oberschenkelhalterung
- 32: Tasche der Betthalterung des Haltegurtes
- 50: Oberschenkelmanschette
- 52: Längsgurt
- 53: langer Quersteg
- 54: Quersteg
- 55: Quersteg
- 56: Beinhalterungsgurt
- 57: Beinhalterungsgurt
- 59: Haltegurt
- 60: Betthalterung

- 61: Betthalterung
- 62: Betthalterung der Oberschenkelmanschette
- 63: Betthalterung des Haltegurtes
- 64: Leibgurt
- 65: gurtartige Bänder
- 66: Öse
- 67: Schulterhalterung
- 68: Schultergurt
- 69: Schultergurt
- 70: Schlaufe
- 71: Schlaufe
- 72: offenes Ende der Schulterhalterung
- 73: geschlossenes Ende der Schulterhalterung
- 74: Öffnung

## Patentansprüche

1. Sicherheitsbandage (100) zur Begrenzung der Beweglichkeit eines auf einem Lager liegenden Patienten bzw. zur Positionierung eines Patienten auf einem Lager mit einem Haltegurt (59), mit dem ein Leibgurt (64), der den Körper des Patienten im Bauchbereich umschließt, beweglich verbunden ist, **dadurch gekennzeichnet, dass** die Sicherheitsbandage (100) mit einer Sicherheitsschlaufe (10) ergänzt ist, die über den Leibgurt (64) und den Haltegurt (59) gesteckt ist, wobei die Sicherheitsschlaufe (10) einseitig zum Kopf- oder Fußende oder beidseitig zum Kopf- und Fußende des Patienten mit mindestens einer materialen Fläche (11) versehen ist, die insbesondere rechteckig ist und die sich in Querrichtung des Lagers erstreckt, wobei die Fläche (11) der festen Anbringung von Fixierelementen, wie Schulterhalterung und Oberschenkelhalterung, und/oder der festen Anbringung von Schlaufen, die der lösbaren Anbringung von Fixierelementen, wie Schulterhalterung und Oberschenkelhalterung, dienlich sind, dient, wobei die Fläche (11) fest oder lösbar an der Sicherheitsbandage (100) befestigt ist.

2. Sicherheitsbandage nach Anspruch 1, **dadurch gekennzeichnet, dass** mindestens eine materiale Fläche (11) über dem Leibgurt (64) und/oder unter dem Haltegurt (59) und/oder zwischen Leibgurt (64) und Haltegurt (59) fest oder lösbar mit diesem(n) verbunden ist.

3. Sicherheitsbandage nach Anspruch 1, **dadurch gekennzeichnet, dass** die Fläche (11) Teil einer Sicherheitsschlaufe (10) ist, die über den Leibgurt (64) und den Haltegurt (59) gesteckt ist, wobei die Schlaufe (10) einseitig zum Kopf- oder Fußende oder beidseitig zum Kopf- und Fußende des Patienten mit der Fläche (11) versehen ist, an denen Fixierelemente und/oder Schlaufen fest angebracht sind, wobei die Schlaufen der lösbaren Anbringung von Fixiermitteln dienen.

4. Sicherheitsbandage nach einem der Ansprüche 1 bis 3, **dadurch gekennzeichnet, dass** die Fläche (11) einlagig oder doppellagig ist.

5. Sicherheitsbandage nach Anspruch 4, **dadurch gekennzeichnet, dass** die Sicherheitsschlaufe (10) einstückig und ringförmig gestaltet ist und zwei Enden aufweist, wobei beide Enden miteinander verbunden sind und eines der Enden der Sicherheitsschlaufe (10) über das andere Ende hinausragt und die einlagige Fläche (11) bildet.

6. Sicherheitsbandage nach Anspruch 4, **dadurch gekennzeichnet, dass** die Sicherheitsschlaufe (10) einstückig und ringförmig gestaltet ist und zwei Enden aufweist, wobei beide Enden miteinander verbunden, vorzugsweise vernäht sind, und beidseitig der Sicherheitsschlaufe (10) die Fläche (11) doppellagig ausgebildet ist.

7. Sicherheitsbandage nach Anspruch 3 oder 4, **dadurch gekennzeichnet, dass** die Sicherheitsschlaufe (10) einstückig und ringförmig gestaltet ist, wobei die Sicherheitsschlaufe (10) außerhalb der einseitigen Fläche (11) zum Ring geschlossen ist.

8. Sicherheitsbandage nach Anspruch 3 oder 4, **dadurch gekennzeichnet, dass** die Sicherheitsschlaufe (10) zwei übereinander angebrachte Stücke (14, 15) aufweist, die verbunden, vorzugsweise vernäht sind und deren Enden die doppellagigen Flächen (11) bilden.

9. Sicherheitsbandage nach Anspruch 8, **dadurch gekennzeichnet, dass** die Stücke (14, 15) unterschiedlich lang sind und ein oder beide Ende(n) des Stücks (15) die einlagige Fläche (11) bildet.

10. Sicherheitsbandage nach einem der Ansprüche 8 oder 9, **dadurch gekennzeichnet, dass** die übereinander aufgebrachten Stücke (14, 15) unterschiedlich breit sind.

11. Sicherheitsbandage nach einem der Ansprüche 3 bis 10, **dadurch gekennzeichnet, dass** die Ränder der Sicherheitsschlaufe (10) zu deren Schutz mit Einfassband eingefasst sind.

12. Sicherheitsbandage nach einem der Ansprüche 3 bis 11, **dadurch gekennzeichnet, dass** die Sicherheitsschlaufe (10) mit Langlöchern (18, 19) versehen ist, durch die die Enden des Haltegurtes (59) und/oder Leibgurtes (64) gesteckt werden können, wodurch eine Sicherung gegen Verrutschen der Schlaufe (10) entlang der Sicherheitsbandage (100) gegeben ist.

13. Sicherheitsbandage nach Anspruch 12, **dadurch gekennzeichnet, dass** das untere der beiden die Sicherheitsschlaufe (10) bildenden Stücke (14, 15) mit Langlöchern (19) versehen ist.

14. Sicherheitsbandage nach Anspruch 12, **dadurch gekennzeichnet, dass** das obere der beiden die Sicherheitsschlaufe (10) bildenden Stücke (14, 15) mit Langlöchern (18) versehen ist.

15. Sicherheitsbandage nach Anspruch 12, **dadurch gekennzeichnet, dass** sowohl das untere als auch das obere der beiden die Sicherheitsschlaufe (10) bildenden Stücke (14, 15) mit Langlöchern (18, 19) versehen sind.

16. Sicherheitsbandage nach einem der Ansprüche 3 bis 12, **dadurch gekennzeichnet, dass** die Sicherheitsschlaufe (10) einstückig ausgebildet ist, wobei die untere Hälfte der Sicherheitsschlaufe gegenüber der oberen Hälfte der Sicherheitsschlaufe (10) verbreitert ist.

17. Sicherheitsbandage nach einem der Ansprüche 13 bis 15, **dadurch gekennzeichnet, dass** das untere Stück (15) an seinen Rändern über je ein Langloch (19) verfügt, durch das der Haltegurt (59) derart gesteckt ist, dass der Haltegurt (59) und die zugehörigen Betthalterungen (63) unterhalb des unteren Stückes (15) verlaufen und somit an den Enden des unteren Stücks (15) befindliche Ösen (66) auf die Schlösser (25) der Betthalterung (63) des Haltegurtes (59) steckbar sind, sodass die Sicherheitsschlaufe gegen Verrutschen auf der Sicherheitsbandage (100) gesichert ist.

18. Sicherheitsbandage nach einem der Ansprüche 13 bis 15, **dadurch gekennzeichnet, dass** das untere Stück (15) an seinen Rändern je über ein Langloch (19) weiter innen, durch das der Haltegurt (59) gesteckt werden kann, und über ein Langloch (20) weiter außen verfügt, durch das nur schmalere Betthalterungen (63) steckbar sind, sodass der Haltegurt (59) zwischen Langloch (19) und Langloch (20) unterhalb des unteren Stückes (15) verläuft, und die Betthalterung (63) durch das Langloch (20) wieder nach oben tritt und am Bettrahmen befestigbar ist, den Haltegurt (59), wobei der Haltegurt (59) breiter als die Betthalterung (63) ausgeführt ist, jedoch zurücklässt, wodurch die Sicherheitsschlaufe gegen Verrutschen auf der Sicherheitsbandage (100) gesichert ist.

19. Sicherheitsbandage nach Anspruch 18, **dadurch gekennzeichnet, dass** der Haltegurt (59) an seinen Rändern verstärkt ist, sodass er nicht biegbar ist, um durch das Langloch (20) gesteckt zu werden.

20. Sicherheitsbandage nach einem der Ansprüche 3 bis 19, **dadurch gekennzeichnet, dass** Nähte von inneren Verbindungsstellen der Flächen (11) mit inneren Laschen (16) zur Nahtsicherung dergestalt gesichert sind, dass die Laschen (16) in Form eines Materialstücks zwischen beiden miteinander vernähten Stücken (14, 15) angeordnet sind.

21. Sicherheitsbandage nach einem der Ansprüche 3 bis 20, **dadurch gekennzeichnet, dass** die Nähte an inneren Verbindungsstellen der Fläche (11) mit Laschen (17) zur Nahtsicherung dergestalt gesichert sind, dass die Lasche (17) in Gestalt eines Materialstücks bei den miteinander vernähten Stücken (14, 15) diese unmittelbar vor der Naht miteinander verbindet.

22. Sicherheitsbandage nach einem der Ansprüche 3 bis 21, **dadurch gekennzeichnet, dass** das eine Ende an der Verbindungsstelle der Fläche (11) nach innen gestülpt ist, womit der nach innen gestülpte Teil der Fläche (11) selbst die Funktion einer inneren Lasche wahrnimmt.

23. Sicherheitsbandage nach Anspruch 22, **dadurch gekennzeichnet, dass** die außen liegende Naht an der Verbindungsstelle an der Fläche (11) mit der Lasche (17) zur Nahtsicherung dergestalt gesichert ist, dass unmittelbar vor der Naht die Lasche (17) die beiden miteinander vernähten Enden der Sicherheitsschlaufe (10) bzw. der die Sicherheitsschlaufe bildenden Stücke (14, 15) miteinander verbindet.

24. Sicherheitsbandage nach einem der vorhergehenden Ansprüche, **dadurch gekennzeichnet, dass** die Sicherheitsschlaufe (10) und die materiale Fläche aus einem nicht biegbaren bzw. kaum biegbaren Material hergestellt ist, sodass der Sicherheitsbandage (100) eine stabile Lagerung innerhalb der Sicherheitsschlaufe (10) gegeben ist.

25. Sicherheitsbandage nach Anspruch 24, **dadurch gekennzeichnet, dass** die Stücke (14, 15) mit Nieten miteinander verbunden sind.

26. Sicherheitsbandage nach einem der Ansprüche 24 oder 25, **dadurch gekennzeichnet, dass** das nicht bzw. kaum biegbare Material in die Sicherheitsschlaufe (10) eingenäht ist, sodass es nicht mit einem Patienten in Berührung kommt.

27. Sicherheitsbandage nach einem der Ansprüche 1 bis 26, **dadurch gekennzeichnet, dass** an der Fläche (11) Schlaufen zur lösbaren Anbringung weiterer Fixiermittel und/oder Fixiermittel fest angebracht sind.

28. Sicherheitsbandage nach einem der vorhergehenden Ansprüche, **dadurch gekennzeichnet, dass** an der Fläche (11) eine Schulterhalterung lösbar angebracht ist.

29. Sicherheitsbandage nach einem der Ansprüchen 1 bis 28, **dadurch gekennzeichnet, dass** an der Fläche (11) eine Schulterhalterung fest angebracht ist.

30. Sicherheitsbandage nach einem der Ansprüche 28 oder 29, **dadurch gekennzeichnet, dass** Schlaufenpaare an den rückwärtigen Schultergurten (68, 69) der Schulterhalterung zur Anbringung weiterer Bettverbindungsgurte, Taillengurte, Kombinationsgurte aus Bettverbindungsgurt und Taillengurt sowie anderer Gurte angebracht sind.

31. Sicherheitsbandage nach Anspruch 30, **dadurch gekennzeichnet, dass** die über die Schlaufenpaare an der Schulterhalterung lösbar anbringbaren Gurte fest an den Schultergurten (68, 69) angebracht sind.

32. Sicherheitsbandage nach einem der Ansprüche 28 bis 31, **dadurch gekennzeichnet, dass** die fest oder lösbar verbundene Schulterhalterung dergestalt gestaltet ist, dass beide Enden (28, 29) des Taillengurtes jeweils mit beiden Schultergurten (68, 69) - diese wiederum miteinander verbindend - lösbar miteinander verbunden sind, wobei sich hierzu an den Enden (28, 29) des Taillengurtes Ösen (66) und an den Schultergurten (68, 69) Schlossaufnahmen befinden.

33. Sicherheitsbandage nach einem der Ansprüche 1 bis 32, **dadurch gekennzeichnet, dass** an der Fläche (11) über lösbar angebrachte Beinhalterungsgurte (56, 57) zwei Oberschenkelmanschetten (50) angebracht sind.

34. Sicherheitsbandage nach einem der Ansprüche 1 bis 32, **dadurch gekennzeichnet, dass** an der Fläche (11) über fest angebrachte Beinhalterungsgurte (56, 57) zwei Oberschenkelmanschetten (50) angebracht sind.

35. Sicherheitsbandage nach einem der Ansprüche 33 oder 34, **dadurch gekennzeichnet, dass** die Oberschenkelmanschetten (50) mit einem Quersteg (55) miteinander verbunden sind.

36. Sicherheitsbandage nach einem der Ansprüche 33 bis 35, **dadurch gekennzeichnet, dass** an den Oberschenkelmanschetten (50) Längsgurte (52) zur lösbaren Verbindung mit dem Leibgurt (64) angebracht sind.

37. Sicherheitsbandage nach einem der Ansprüche 33 bis 35, **dadurch gekennzeichnet, dass** an den Oberschenkelmanschetten (50) Längsgurte (52) zur festen Verbindung mit dem Leibgurt (64) angebracht sind.

38. Sicherheitsbandage nach einem der Ansprüche 36 oder 37, **dadurch gekennzeichnet, dass** die Beinhalterungsgurte (56, 57) mit den jeweils darüber liegenden Längsgurten (52) und die Beinhalterungsgurte (56, 57) wiederum miteinander mit einem langen Quersteg (53) verbunden werden, dessen Enden die Oberschenkel umgreifen.

39. Sicherheitsbandage nach einem der Ansprüche 33 bis 38, **dadurch gekennzeichnet, dass** der Raum zwischen Sicherheitsschlaufe (10), Beinhalterungsgurten (56, 57) und Querstegen (53, 54, 55) flächig ausgestaltet ist oder die Gurte selbst oder in Verbindung mit weiteren Gurten eine solche Flächenfunktion bildend angeordnet sind, dass das Gesäß des Patienten nicht zwischen den Beinhalterungsgurten (56, 57) hindurchrutschen kann.

40. Sicherheitsbandage nach einem der Ansprüche 33 bis 39, **dadurch gekennzeichnet, dass** die Oberschenkelmanschette (50) mit einer zusätzlichen einen Bettverbindungsgurt bildenden Betthalterung (62) zur zusätzlichen lösbaren Befestigung am Bettrahmen versehen ist.

41. Sicherheitsbandage nach einem der Ansprüche 33 bis 40, **dadurch gekennzeichnet, dass** an den Enden der Betthalterungen (62) der Oberschenkelmanschetten (50) jeweils eine Schlaufe angebracht ist, durch die die Betthalterung (63) des Haltegurts (59) durchsteckbar ist.

42. Sicherheitsbandage nach einem der Ansprüche 33 bis 40, **dadurch gekennzeichnet, dass** die Betthalterung (62) der Oberschenkelmanschette (50) ohne weitere Verschlussmittel direkt mit den Betthalterungen (63) des Haltegurts (59) fest verbunden sind oder mit dem Haltegurt (59) selbst fest verbunden sind, etwa vernäht oder vernietet.

43. Sicherheitsbandage nach einem der Ansprüche 33 bis 40, **dadurch gekennzeichnet, dass** die Oberschenkelmanschette (50) mit zusätzlichen Bettverbindungsgurte bildenden Betthalterungen (62) zur zusätzlichen Befestigung an den Gurtschlössern der Betthalterungen (63) des Haltegurtes (59) versehen ist.

44. Sicherheitsbandage nach einem der Ansprüche 27 bis 43, **dadurch gekennzeichnet, dass** an einer oder beiden der Flächen (11) eine Schulterhalterung und/oder an einer der Flächen über Beinhalterungsgurte (56, 57) Oberschenkelmanschetten (50) fest und/oder lösbar angebracht sind.

45. Sicherheitsbandage nach vorhergehenden Ansprüchen, **dadurch gekennzeichnet, dass** die Sicherheitsschlaufe (10) und/oder die materiale Fläche mit der Sicherheitsbandage (100) fest verbunden ist, etwa vernäht oder mit Niete.

46. Sicherheitsbandage nach einem der vorhergehenden Ansprüche, **dadurch gekennzeichnet, dass** die materiale Fläche oder die Sicherheitsschlaufe (10) unter Beibehaltung der Leibgurt (64) und Haltegurt (59) zusammenhaltenden gurtartigen Bänder (65) nur mit dem Leibgurt (64) der Sicherheitsbandage (100) verbunden ist.

47. Sicherheitsbandage nach einem der vorhergehenden Ansprüche, **dadurch gekennzeichnet, dass** die materiale Fläche oder die Sicherheitsschlaufe (10) unter Beibehaltung der Leibgurt (64) und Haltegurt (59) zusammenhaltenden gurtartigen Bänder (65) nur mit dem Haltegurt (59) der Sicherheitsbandage (100) verbunden ist.

48. Sicherheitsbandage nach einem der vorherigen Ansprüche, **dadurch gekennzeichnet, dass** die Fixiermittel mit Gurtschlössern gesichert sind.

49. Sicherheitsbandage nach einem der vorherigen Ansprüche, **dadurch gekennzeichnet, dass** die Fixiermittel mit Klettverschlüssen, Gürtelschnallen und dergleichen gesichert sind.

50. Sicherheitsbandage nach einem der Ansprüche 3 bis 49, **dadurch gekennzeichnet, dass** die Sicherheitsschlaufe (10) mindestens zwei abstehende Schlitze (22, 23) aufweist, um an der Sicherheitsbandage befindliche Schlaufen hindurch zu stecken, damit trotz übergesteckter Sicherheitsschlaufe (10) an den Schlaufen der Sicherheitsbandage lösbare Fixierelemente angebracht werden können.

51. Nachrüstsatz für eine Sicherheitsbandage (100) zur Begrenzung der Beweglichkeit eines auf einem Lager liegenden Patienten bzw. zur Positionierung eines Patienten auf einem Lager mit einem Haltegurt (59) mit dem ein Leibgurt (64), der den Körper des Patienten im Bauchbereich umschließt, beweglich verbunden ist, umfassend eine Sicherheitsschlaufe (10), die über den Leibgurt (64) und den Haltegurt (59) gesteckt werden kann, wobei die Sicherheitsschlaufe (10) einseitig zum Kopf- oder Fußende oder beidseitig zum Kopf- und Fußende des Patienten mit mindestens einer materialen Fläche (11) versehen ist, die insbesondere rechteckig ist und sich in Querrichtung des Lagers erstreckt, wobei die Fläche (11) der festen Anbringung von Fixiermitteln, wie Schulterhalterung und Oberschenkelhalterung und/oder der festen Anbringung von Schlaufen, die der lösbaren Anbringung von Fixiermitteln, wie Schulterhalterung und Oberschenkelhalterung, dienlich sind, dient, wobei die Fläche (11) fest oder lösbar an der Sicherheitsbandage (100) anbringbar ist.

52. Nachrüstsatz nach Anspruch 50, **dadurch gekennzeichnet, dass** die Sicherheitsbandage (100) nach einem der Ansprüche 2 bis 49 ausgebildet ist.

## Claims

1. A safety brace (100) for limiting the mobility of a patient lying on a bed or for positioning a patient on a bed, respectively, by means of a harness (59), to which a body belt (64), which surrounds the body of the patient in the abdominal area, is movably connected, **characterized in that** the safety brace (100) is supplemented with a safety strap (10), which is tucked across the body belt (64) and the harness (59), wherein the safety strap (10) is provided on one side towards the head or foot end or on both sides towards the head and foot end of the patient with at least one material surface (11), which is in particular rectangular and which extends in transverse direction of the bed, wherein the surface (11) serves for fixedly attaching fixing elements, such as a shoulder restraint and a thigh restraint and/or for fixedly attaching straps, which are useful for detachably attaching fixing elements, such as a shoulder restraint and a thigh restraint, wherein the surface (11) is fixedly or detachably fastened to the safety brace (100).

2. The safety brace according to claim 1, **characterized in that** at least one material surface (11) above the body belt (64) and/or below the harness (59) and/or between body belt (64) and harness (59) is fixedly or detachably connected thereto.

3. The safety brace according to claim 1, **characterized in that** the surface (11) is part of a safety strap (10), which is tucked across the body belt (64) and the harness (59), wherein the strap (10) is provided on one side towards the head or foot end or on both sides towards the head and foot end of the patient with the surface (11), to which fixing elements and/or straps are fixedly attached, wherein the straps serve for detachably attaching fixing elements.

4. The safety brace according to one of claims 1 to 3, **characterized in that** the surface (11) is one-layered or two-layered.

5. The safety brace according to claim 4, **characterized in that** the safety strap (10) is designed integrally and in a ring-shaped manner and encompasses two ends, wherein both ends are connected to one another and one of the ends of the safety strap (10) projects beyond the other end and forms the one-layered surface (11).

6. The safety brace according to claim 4, **characterized in that** the safety brace (10) is designed integrally and in a ring-shaped manner and encompasses two ends, wherein both ends are connected to one another, preferably by means of sewing, and the surface (11) on both sides of the safety strap (10) is embodied so as to be two-layered.

7. The safety brace according to claim 3 or 4, **characterized in that** the safety strap (10) is designed integrally and in a ring-shaped manner, wherein the safety strap (10) is closed outside of the unilateral surface (11) to the ring.

8. The safety brace according to claim 3 or 4, **characterized in that** the safety strap (10) encompasses two parts (14, 15) attached on top of one another, which are connected, preferably by means of sewing, and the ends of which form the two-layered surfaces (11).

9. The safety brace according to claim 8, **characterized in that** the parts (14, 15) have different lengths and one or both end(s) of the part (15) form(s) the one-layered surface (11).

10. The safety brace according to one of claims 8 or 9, **characterized in that** the parts (14, 15), which are applied on top of one another, have different widths.

11. The safety brace according to one of claims 3 to 10, **characterized in that** the edges of the safety strap (10) are surrounded by bordering tape for the protection thereof.

12. The safety brace according to one of claims 3 to 11, **characterized in that** the safety strap (10) is provided with elongated holes (18, 19), through which the ends of the harness (59) and/or body belt (64) can be inserted, thus providing protection against a slipping of the strap (10) along the safety brace (100).

13. The safety brace according to claim 12, **characterized in that** the lower one of the two parts (14, 15), which forms the safety strap (10), is provided with elongated holes (19).

14. The safety brace according to claim 12, **characterized in that** the upper one of the two parts (14, 15), which forms the safety strap (10), is provided with elongated holes (18).

15. The safety brace according to claim 12, **characterized in that** the lower as well as the upper one of the two parts (14, 15), which form the safety strap (10), are provided with elongated holes (18, 19).

16. The safety brace according to one of claims 3 to 12, **characterized in that** the safety strap (10) is embodied integrally, wherein the lower half of the safety strap is widened as compared to the upper half of the safety strap (10).

17. The safety brace according to one of claims 13 to 15, **characterized in that**, at its edges, the lower part (15) in each case has an elongated hole (19), through which the harness (59) is inserted such that the harness (59) and the corresponding bed mounts (63) run below the lower part (15) and eyelets (66) located at the ends of the lower part (15) can thus be plugged into the locks (25) of the bed mount (63) of the harness (59), so that the safety strap is secured against slipping on the safety brace (100).

18. The safety brace according to one of claims 13 to 15, **characterized in that** the lower part (15), at its edges, in each case has an elongated hole (19) further on the inside, through which the harness (59) can be inserted, and an elongated hole (20) further on the outside, through which only narrower bed mounts (63) can be inserted, so that the harness (59) between elongated hole (19) and elongated hole (20) runs below the lower part (15), and the bed mount (63) goes upwards again through the elongated hole (20) and can be fastened to the bed frame, but leaves behind the harness (59), wherein the harness (59) is embodied so as to be wider than the bed mount (63), thus securing the safety strap against slipping on the safety brace (100).

19. The safety brace according to claim 18, **characterized in that** the harness (59) is reinforced at its edges, so that it is not bendable, so as to be inserted through the elongated hole (20).

20. The safety brace according to one of claims 3 to 19, **characterized in that** seams of inner joints of the surfaces (11) are secured by means of inner tabs (16) for securing the seam such that the tabs (16) are arranged in the form of a material piece between both parts (14, 15), which are sewn together.

21. The safety brace according to one of claims 3 to 20, **characterized in that** the seams on inner joints of the surface (11) are secured by means of tabs (17) for securing the seam such that the tab (17) in the form of a material piece in the case of the parts (14, 15), which are sewn to one another, connects them to one another directly in front of the seam.

22. The safety brace according to one of claims 3 to 21, **characterized in that** the one end on the joint of the surface (11) is folded inwardly, whereby the part of the surface (11), which has been folded inwardly, in turn assumes the function of an inner tab.

23. The safety brace according to claim 22, **characterized in that** the seam located on the outside at the joint is secured to the surface (11) by means of the tab (17) for securing the seam such that the tab (17) connects the two ends of the safety strap (10) or of the parts (14, 15) forming the safety strap, respectively, which have been sewn to one another, to one another directly in front of the seam.

24. The safety brace according to one of the preceding claims, **characterized in that** the safety strap (10) and the material surface is made of a material, which cannot be bent or which can only be bent slightly, respectively, so that the safety brace (100) is provided with a stable support within the safety strap (10).

25. The safety brace according to claim 24, **characterized in that** the parts (14, 15) are connected to one another by means of rivets.

26. The safety brace according to one of claims 24 or 25, **characterized in that** the material, which cannot be bent or which can only be bent slightly, respectively, is sewn into the safety strap (10), so that it does not come into contact with a patient.

27. The safety brace according to one of claims 1 to 26, **characterized in that** straps for detachably attaching further fixing elements and/or fixing elements are fixedly attached to the surface (11).

28. The safety brace according to one of the preceding claims, **characterized in that** a shoulder restraint is detachably attached to the surface (11).

29. The safety brace according to one of claims 1 to 28, **characterized in that** a shoulder restraint is fixedly attached to the surface (11).

30. The safety brace according to one of claims 28 or 29, **characterized in that** pairs of straps are attached to the rear shoulder belts (68, 69) of the shoulder restraint for attaching further bed connecting belts, waist belts, combination belts of bed connecting belt and waist belt as well as other belts.

31. The safety brace according to claim 30, **characterized in that** the belts, which can be detachably attached to the shoulder restraint via the pairs of straps, are fixedly attached to the shoulder belts (68, 69).

32. The safety brace according to one of claims 28 to 31, **characterized in that** the shoulder restraint, which is fixedly or detachably connected, is designed such that both ends (28, 29) of the waist belt are detachably connected to one other in each case by means of both shoulder belts (68, 69) - in turn connecting them to one another, wherein eyelets (66) are located for this purpose on the ends (28, 29) of the waist belt and lock accommodations are located on the shoulder belts (68, 69).

33. The safety brace according to one of claims 1 to 32, **characterized in that** two thigh cuffs (50) are attached to the surface (11) via leg restraint belts (56, 57), which are detachably attached.

34. The safety brace according to one of claims 1 to 32, **characterized in that** two thigh cuffs (50) are attached to the surface (11) via fixedly attached leg restraint belts (56, 57).

35. The safety brace according to one of claims 33 or 34, **characterized in that** the thigh cuffs (50) are connected to one another by means of a cross bar (55).

36. The safety brace according to one of claims 33 to 35, **characterized in that** longitudinal belts (52) are attached to the thigh cuffs (50) for detachably connecting to the body belt (64).

37. The safety brace according to one of claims 33 to 35, **characterized in that** longitudinal belts (52) are attached to the thigh cuffs (50) for fixedly connecting to the body belt (64).

38. The safety brace according to one of claims 36 or 37, **characterized in that** the leg restraint belts (56, 57) are connected to the longitudinal belts (52), which are in each case located thereabove, and the leg restraint belts (56, 57) are in each case in turn connected together to a long cross bar (53), the ends of which encompass the thighs.

39. The safety brace according to one of claims 33 to 38, **characterized in that** the space between safety strap (10), leg restraint belts (56, 57) and cross bars (53, 54, 55) is embodied to be flat or the belts in turn or in connection with further belts are arranged so as to form such a surface function that the buttocks of the patient cannot slip through between the leg restraint belts (56, 57).

40. The safety brace according to one of claims 33 to 39, **characterized in that** the thigh cuff (50) is provided with an additional bed mount (62), which forms a bed connecting belt, for additionally detachably fastening to the bed frame.

41. The safety brace according to one of claims 33 to 40, **characterized in that** a strap, through which the bed mount (63) of the harness (59) can be inserted, is in each case attached to the ends of the bed mounts (62) of the thigh cuffs (50).

42. The safety brace according to one of claims 33 to 40, **characterized in that** the bed mount (62) of the thigh cuff (50) is fixedly connected directly to the bed mounts (63) of the harness (59) without further locking means or are fixedly connected to the harness (59) in turn, for instance by means of sewing or riveting.

43. The safety brace according to one of claims 33 to 40, **characterized in that** the thigh cuff (50) is provided with additional bed mounts (62) forming bed connecting belts for additionally fastening to the belt locks of the bed mounts (63) of the harness (59).

44. The safety brace according to one of claims 27 to 43, **characterized in that** a shoulder restraint is fixedly and/or detachably attached to one or both of the surfaces (11) and/or that thigh cuffs (50) are fixedly and/or detachably attached to one of the surfaces via leg restraint belts (56, 57).

45. The safety brace according to the preceding claims, **characterized in that** the safety strap (10) and/or the material surface is fixedly connected to the safety brace (100), for instance by means of sewing or by means of a rivet.

46. The safety brace according to one of the preceding claims, **characterized in that** the material surface or the safety strap (10) is only connected to the body belt (64) of the safety brace (100) by maintaining the belt-like straps (65), which hold the body belt (64) and harness (59) together.

47. The safety brace according to one of the preceding claims, **characterized in that** the material surface or the safety strap (10) is only connected to the harness (59) of the safety brace (100) while maintaining the belt-like straps (65), which hold the body belt (64) and the harness (59) together.

48. The safety brace according to one of the preceding claims, **characterized in that** the fixing elements are secured by means of belt locks.

49. The safety brace according to one of the preceding claims, **characterized in that** the fixing elements are secured by means of adhesive closures, belt buckles and the like.

50. The safety brace according to one of claims 3 to 49, **characterized in that** the safety strap (10) encompasses at least two slits (22, 23), which stick out, so as to be inserted through the straps located on the safety brace, so that detachable fixing elements can be attached to the straps of the safety brace in spite of the safety strap (10) being inserted thereabove.

51. An upgrade kit for a safety brace (100) for limiting the movability of a patient lying on a bed or for positioning a patient on a bed, respectively, by means of a harness (59), to which a body belt (64), which surrounds the body of the patient in the abdominal area, is movably connected, comprising a safety strap (10), which can be tucked across the body belt (64) and the harness (59), wherein the safety strap (10) is provided on one side towards the head or foot end or on both sides towards the head and foot end of the patient with at least one material surface (11), which is in particular rectangular and which extends in transverse direction of the bed, wherein the surface (11) serves for fixedly attaching fixing elements, such as a shoulder restraint and a thigh restraint and/or for fixedly attaching straps, which are useful for detachably attaching fixing elements, such as a shoulder restraint and a thigh restraint, wherein the surface (11) can be fixedly or detachably fastened to the safety brace (100).

52. The upgrade kit according to claim 50, **characterized in that** the safety brace (100) is embodied according to one of claims 2 to 49.

## Revendications

1. Bandage de sécurité (100) permettant de limiter la mobilité d'un patient couché sur une couchette ou de positionner un patient sur une couchette, comportant une sangle de retenue (59) à laquelle une sangle corporelle (64) entourant le patient au niveau du ventre est reliée de manière mobile, **caractérisé en ce que** le bandage de sécurité (100) est complété d'une boucle de sécurité (10) qui est fichée sur la sangle corporelle (64) et la sangle de retenue (59), la boucle de sécurité (10) étant pourvue unilatéralement vers l'extrémité de la tête ou des pieds ou bilatéralement vers l'extrémité de la tête ou des pieds du patient d'au moins une surface de matériau (11) est en particulier rectangulaire et qui s'étend dans le sens transversal de la couchette, la surface (11) servant à l'installation fixe d'éléments de fixation, comme une fixation d'épaule ou une fixation de cuisse, et/ou à la fixation fixe de boucles qui sont utiles pour y installer de manière amovible des éléments de fixation comme une fixation d'épaule ou une fixation de cuisse, la surface (11) étant fixée de manière fixe ou amovible au bandage de sécurité (100).

2. Bandage de sécurité selon la revendication 1, **caractérisé en ce qu'**au moins une surface de matériau (11) est reliée par la sangle corporelle (64) et/ou la sangle de retenue (59) et/ou la sangle corporelle (64) et la sangle de retenue (59) de manière fixe ou dissociable à celle(s)-ci

3. Bandage de sécurité selon la revendication 1, **caractérisé en ce que** la surface (11) fait partie d'une boucle de sécurité (10) qui est fichée sur la sangle corporelle (64) et la sangle de retenue (59), la boucle (10) étant pourvue unilatéralement vers l'extrémité de la tête ou des pieds ou bilatéralement vers l'extrémité de la tête ou des pieds du patient de la surface (11) à laquelle des éléments de fixation et/ou des boucles sont installés de manière fixe, les boucles servant à la fixation amovible de moyens de fixation.

4. Bandage de sécurité selon une des revendications 1 à 3, **caractérisé en ce que** la surface (11) a une épaisseur simple ou double.

5. Bandage de sécurité selon la revendication 4, **caractérisé en ce que** la boucle de sécurité (10) est conformée en un bloc et en forme annulaire et présente deux extrémités, les deux extrémités étant reliées entre elles et une des extrémités de la boucle de sécurité (10) dépassant de l'autre extrémité et constituant la surface à simple épaisseur (11).

6. Bandage de sécurité selon la revendication 4, **caractérisé en ce que** la boucle de sécurité (10) est conformée en un bloc et en forme annulaire et présente deux extrémités, les deux extrémités étant reliées entre elles, de préférence cousues, et la surface (11) étant réalisée en double épaisseur des deux côtés de la boucle de sécurité (10).

7. Bandage de sécurité selon la revendication 3 ou 4, **caractérisé en ce que** la boucle de sécurité (10) est conformée en un bloc et en forme annulaire, la boucle de sécurité (10) étant fermée vers l'anneau en dehors de la surface unilatérale (11).

8. Bandage de sécurité selon la revendication 3 ou 4, **caractérisé en ce que** la boucle de sécurité (10) présente deux pièces superposées (14, 15) qui sont reliées, de préférence cousues, et dont les extrémités constituent les surfaces à double épaisseur (11).

9. Bandage de sécurité selon la revendication 8, **caractérisé en ce que** les pièces (14, 15) sont de longueurs différentes et qu'une extrémité ou les deux extrémités de la pièce (15) constitue la surface à simple épaisseur (11).

10. Bandage de sécurité selon une des revendications 8 ou 9, **caractérisé en ce que** les pièces superposées (14, 15) sont de largeurs différentes.

11. Bandage de sécurité selon une des revendications 3 à 10, **caractérisé en ce que** les bords de la boucle de sécurité (10) sont pris dans une bande de bordage pour les protéger.

12. Bandage de sécurité selon une des revendications 3 à 11, **caractérisé en ce que** la boucle de sécurité (10) est pourvue de trous oblongs (18, 19) dans lesquels peuvent être fichées les extrémités de la sangle de retenue (59) et/ou de la sangle corporelle (64), ce qui crée une sécurité contre le glissement de la boucle (10) le long du bandage de sécurité (100).

13. Bandage de sécurité selon la revendication 12, **caractérisé en ce que** la pièce inférieure sur les deux pièces (14, 15) formant la boucle de sécurité (10) est pourvue de trous oblongs (19).

14. Bandage de sécurité selon la revendication 12, **caractérisé en ce que** la pièce supérieure sur les deux pièces (14, 15) formant la boucle de sécurité (10) est pourvue de trous oblongs (18).

15. Bandage de sécurité selon la revendication 12, **caractérisé en ce qu'**aussi bien la pièce inférieure que la pièce supérieure sur les deux pièces (14, 15) formant la boucle de sécurité (10) sont pourvues de trous oblongs (18, 19).

16. Bandage de sécurité selon une des revendications 3 à 1, **caractérisé en ce que** la boucle de sécurité (10) est réalisée en un bloc, la moitié inférieure de la boucle de sécurité étant évasée par rapport à la moitié supérieure de la boucle de sécurité (10).

17. Bandage de sécurité selon une des revendications 13 à 15, **caractérisé en ce que** la pièce inférieure (15) dispose à ses bords chacune d'un trou oblong (19) dans lequel la sangle de retenue (59) est fichée de manière à ce que la sangle de retenue (59) et les fixations de lit correspondantes (63) s'étendent en-dessous de la pièce inférieure (15) et puissent ainsi être fichées dans les oeillets (66) se trouvant aux extrémités de la pièce inférieure (15) sur les cadenas (25) de la fixation de lit (63) de la sangle de retenue (59), de sorte que la boucle de sécurité est bloquée en évitant tout glissement sur le bandage de sécurité (100).

18. Bandage de sécurité selon une des revendications 13 à 15, **caractérisé en ce que** la pièce inférieure (15) dispose à ses bords chacune d'un trou oblong (19) plus loin vers l'intérieur, dans lequel la sangle de retenue (59) peut être fichée, et d'un trou oblong (20) plus loin vers l'extérieur, dans lequel seules des fixations de lit plus étroites (63) peuvent être fichées, de sorte que la sangle de retenue (59) s'étend entre le trou oblong (19) et le trou oblong (20) en-dessous de la pièce inférieure (15), et que la fixation de lit (63) remonte à travers le trou oblong (20) et peut être fixée au cadre du lit, la sangle de retenue (59) étant conformée plus large que la fixation de lit (63) en laissant toutefois derrière la sangle de retenue (59), la sangle de retenue (59) étant réalisée plus large que la fixation de lit (63) mais allant en diminuant, ce qui a pour effet que la boucle de sécurité est bloquée en évitant tout glissement sur le bandage de sécurité (100).

19. Bandage de sécurité selon la revendication 18, **caractérisé en ce que** la sangle de retenue (59) est renforcée sur ses bords de manière à ne pas être flexible, afin de pouvoir être fichée dans le trou oblong (20).

20. Bandage de sécurité selon une des revendications 3 à 19, **caractérisé en ce que** les coutures des points de liaison intérieurs des surfaces (11) sont renforcées par des pattes intérieures (16) de renforcement des coutures, de sorte que les pattes (16) sont disposées sous forme d'une pièce de matériau entre les deux pièces cousues ensemble (14, 15).

21. Bandage de sécurité selon une des revendications 3 à 20, **caractérisé en ce que** les coutures sont renforcées aux points de liaison intérieurs de la surface (11) par des pattes (17) de renforcement des coutures, de sorte que la patte (17) se présentant sous la forme d'une pièce de matériau dans les deux pièces cousues ensemble (14, 15) relie celles-ci entre elles juste avant la couture.

22. Bandage de sécurité selon une des revendications 3 à 21, **caractérisé en ce qu'**une extrémité est emboutie vers l'intérieur au point de liaison de la surface (11), la pièce emboutie vers l'intérieur de la surface (11) assurant elle-même la fonction d'une patte intérieure.

23. Bandage de sécurité selon la revendication 22, **caractérisé en ce que** la couture placée à l'extérieur est renforcée au point de liaison avec la surface (11) par la patte (17) de renforcement de couture de manière à ce que, juste avant la couture, la patte (17) relie entre elles les deux extrémités cousues ensemble de la boucle de sécurité (10) ou les pièces (14, 15) formant la boucle de sécurité.

24. Bandage de sécurité selon une des revendications précédentes, **caractérisé en ce que** la boucle de sécurité (10) et la surface de matériau sont faites d'un matériau non flexible ou à peine flexible, de sorte que le bandage de sécurité (100) bénéficie d'un appui stable dans la boucle de sécurité (10).

25. Bandage de sécurité selon la revendication 24, **caractérisé en ce que** les pièces (14, 15) sont reliées entre elles par des rivets.

26. Bandage de sécurité selon une des revendications 24 ou 25, **caractérisé en ce que** le matériau non ou à peine flexible est cousu dans la boucle de sécurité (10) de manière à ne pas venir en contact avec un patient.

27. Bandage de sécurité selon une des revendications 1 à 26, **caractérisé en ce que**, sur la surface (11), sont installées fixement des boucles permettant l'installation amovible d'autres moyens de fixations et/ou de moyens de fixation.

28. Bandage de sécurité selon une des revendications précédentes, **caractérisé en ce que**, sur la surface (11), est installée de manière amovible une fixation d'épaule.

29. Bandage de sécurité selon une des revendications 1 à 28, **caractérisé en ce que**, sur la surface (11), est installée de manière fixe une fixation d'épaule.

30. Bandage de sécurité selon une des revendications 28 ou 29, **caractérisé en ce que** des paires de boucles sont installées sur les sangles d'épaule arrière (68, 69) de la fixation d'épaule pour y installer d'autres sangles de liaison au lit, ceintures de taille, combinés sangles de liaison au lit et ceintures de taille et d'autres sangles.

31. Bandage de sécurité selon la revendication 30, **caractérisé en ce que** les sangles pouvant être installées de manière amovible à l'aide des paires de boucles à la la fixation d'épaule sont installées de manière fixe sur les sangles d'épaules (68, 69).

32. Bandage de sécurité selon une des revendications 28 à 31, **caractérisé en ce que** la fixation d'épaule reliée de manière fixe ou amovible est conçue de manière à ce que les deux extrémités (28, 29) de la ceinture de taille comportant respectivement les deux sangles d'épaule (68, 69) - en reliant à nouveau celles-ci entre elles - soient respectivement reliées entre elles de manière amovible, des oeillets (66) se trouvant à cet effet aux extrémités (28, 29) de la ceinture de taille et des supports de cadenas sur les sangles d'épaules (68, 69).

33. Bandage de sécurité selon une des revendications 1 à 32, **caractérisé en ce que**, sur la surface (11), deux manchons de cuisse (50) sont installés à l'aide de sangles de rétention installées de manière amovible (56, 57).

34. Bandage de sécurité selon une des revendications 1 à 32, **caractérisé en ce que**, sur la surface (11), deux manchons de cuisses (50) sont installés à l'aide de sangles de rétention installées de manière fixe (56, 57).

35. Bandage de sécurité selon une des revendications 33 ou 34, **caractérisé en ce que** les manchons de cuisses (50) sont reliés entre eux par une traverse (55).

36. Bandage de sécurité selon une des revendications 33 à 35, **caractérisé en ce que**, sur les manchons de cuisses (50), sont installées des sangles longitudinales (52) permettant une liaison amovible avec la sangle corporelle (64).

37. Bandage de sécurité selon une des revendications 33 à 35, **caractérisé en ce que**, sur les manchons de cuisses (50), sont installées des sangles longitudinales (52) permettant une liaison fixe avec la sangle corporelle (64).

38. Bandage de sécurité selon une des revendications 36 ou 37, **caractérisé en ce que** les sangles de rétention (56, 57), avec les sangles longitudinales (52) reposant respectivement dessus, et les sangles de rétention (56, 57) sont à leur tour reliées entre elles par une longue traverse (53) dont les extrémités entourent les cuisses.

39. Bandage de sécurité selon une des revendications 33 à 38, **caractérisé en ce que** l'espace entre la boucle de sécurité (10), les sangles de rétention (56, 57) et les traverses (53, 54, 55) a une conformation plane ou que les sangles sont disposées de manière à assurer elles-mêmes ou en liaison avec d'autres sangles une fonction de surface de manière à ce que le postérieur du patient ne puisse pas glisser entre les sangles de rétention (56, 57).

40. Bandage de sécurité selon une des revendications 33 à 39, **caractérisé en ce que** le manchon de cuisse (50) est pourvu d'une fixation de lit (62) formant une sangle de liaison au lit supplémentaire pour la fixation amovible supplémentaire au cadre du lit.

41. Bandage de sécurité selon une des revendications 33 à 40, **caractérisé en ce que**, aux extrémités des fixations de lit (62) des manchons de cuisses (50), est installée respectivement une boucle dans laquelle la fixation de lit (63) de la sangle de retenue (59) peut être fichée.

42. Bandage de sécurité selon une des revendications 33 à 40, **caractérisé en ce que** la fixation de lit (62) du manchon de cuisse (50) ou d'autres moyens de fermeture sont reliés directement aux fixations de lit (63) de la sangle de retenue (59) ou sont reliés de manière fixe à la sangle de retenue (59) elle-même, par exemple cousus ou rivetés.

43. Bandage de sécurité selon une des revendications 33 à 40, **caractérisé en ce que** la sangle de cuisse (50) est pourvue de fixations de lit (62) formant des sangles de liaison au lit supplémentaires pour la fixation supplémentaire aux cadenas de sangle des fixations de lit (63) de la sangle de retenue (59).

44. Bandage de sécurité selon une des revendications 27 à 43, **caractérisé en ce que**, sur une surface ou les deux (11), une fixation d'épaule et/ou sur une des surfaces, des manchons de cuisses (50) sont fixés de manière fixe et/ou amovible via des sangles de contention (56, 57).

45. Bandage de sécurité selon une des revendications précédentes, **caractérisé en ce que** la boucle de sécurité (10) et/ou la surface de matériau sont reliées de manière fixe au bandage de sécurité (100), par exemple cousues ou rivetées.

46. Bandage de sécurité selon une des revendications précédentes, **caractérisé en ce que** la surface de matériau ou la boucle de sécurité (10) est reliée, en conservant des bandes de type sangle (65) maintenant ensemble la sangle corporelle (64) et la sangle de retenue (59), seulement à la sangle corporelle (64) du bandage de sécurité (100).

47. Bandage de sécurité selon une des revendications précédentes, **caractérisé en ce que** la surface de matériau ou la boucle de sécurité (10) est reliée, en conservant des bandes de type sangle (65) maintenant ensemble la sangle corporelle (64) et la sangle de retenue (59), seulement à la sangle de retenue (59) du bandage de sécurité (100).

48. Bandage de sécurité selon une des revendications précédentes, **caractérisé en ce que** les moyens de fixation sont bloqués par des cadenas de sangles.

49. Bandage de sécurité selon une des revendications précédentes, **caractérisé en ce que** les moyens de fixation sont bloqués par des fermetures agrippantes, des boucles de ceinture et similaires.

50. Bandage de sécurité selon une des revendications 3 à 49, **caractérisé en ce que** la boucle de sécurité (10) présente au moins deux fentes (22, 23) proéminentes pour ficher les boucles se trouvant sur le bandage de sécurité afin que, bien qu'une boucle de sécurité (10) soit fichée par-dessus, des éléments de fixation amovibles puissent être fixés aux boucles du bandage de sécurité.

51. Kit de complément pour bandage de sécurité (100) permettant de limiter la mobilité d'un patient couché sur une couchette ou de positionner un patient sur une couchette, comportant une boucle de sécurité (10) à laquelle une sangle corporelle (64) entourant le corps du patient au niveau du ventre est reliée de manière mobile, comprenant une boucle de sécurité (10) qui peut être fichée sur la sangle corporelle (64) et la sangle de retenue (59), la boucle de sécurité (10) étant pourvue unilatéralement vers l'extrémité de la tête ou des pieds ou bilatéralement vers l'extrémité de la tête ou des pieds du patient d'au moins une surface de matériau (11) qui est en particulier rectangulaire et qui s'étend dans le sens transversal de la couchette, la surface (11) servant à l'installation fixe d'éléments de fixation, comme une fixation d'épaule ou une fixation de cuisse, et/ou à l'installation fixe de boucles qui sont utiles pour y installer de manière amovible des éléments de fixation comme une fixation d'épaule ou une fixation de cuisse, la surface (11) pouvant être fixée de manière fixe ou amovible au bandage de sécurité (100).

52. Kit de complément selon la revendication 50, **caractérisé en ce que** le bandage de sécurité (100) est réalisé selon une des revendications 2 à 49.
